# EUROPEAN PATENT APPLICATION

(11) **EP 4 624 640 A1**
(43) Date of publication of application: **01.10.2025**
(21) Application number: 24167882.0
(22) Date of filing: 29.03.2024
(51) Int. Cl.: D01F 1/10, A23L 13/00, C12N 5/00, C12N 11/04, D01F 4/00, D01F 8/00, D01F 8/02, D01F 8/18

(54) **PRODUCING CELL CONTAINING FIBERS FOR ARTIFICIAL MEAT**

(71) Applicant: DWI - Leibniz-Institut für Interaktive Materialien e.V., 52074 Aachen (DE)
(72) Inventor: Omidinia Anarkoli, Rahman, 52074 Aachen (DE); De Laporte, Laura, 52074 Aachen (DE)
(74) Representative: Rose, Jonas Christopher

(57) **Abstract**

The present invention relates to the field of tissue engineering, particularly the field of artificial meat production. The present invention provides a method for producing a cell-containing fiber comprising, providing a microcarrier, cells capable of interacting with the microcarrier, and a composition comprising a compound capable of crosslinking or solidifying, which are spun to generate a fiber, which is at least partially crosslinked or solidified. The present invention further provides cell-containing fibers. The present invention further provides an edible, cell-containing fiber for use as artificial meat. The present invention further provides a spinning system for producing a cell-containing core-shell hollow fiber. The present invention further provides a method for producing artificial meat. The invention further provides artificial meat comprising assembled and/or collected one or more edible, cell-containing fiber(s).

## Description

### TECHNICAL FIELD OF THE INVENTION

The present invention relates to the field of tissue engineering, particularly the field of artificial meat production. The present invention provides a method for producing a cell-containing fiber comprising, providing a microcarrier, cells capable of interacting with the microcarrier, and a composition comprising a compound capable of crosslinking or solidifying, which are spun to generate a fiber, which is at least partially crosslinked or solidified. The present invention further provides cell-containing fibers. The present invention further provides an edible, cell-containing fiber for use as artificial meat. The present invention further provides a spinning system for producing a cell-containing core-shell hollow fiber. The present invention further provides a method for producing artificial meat. The present invention further provides artificial meat comprising assembled and/or collected one or more edible, cell-containing fiber(s).

### BACKGROUND OF THE INVENTION

The world's population has surpassed the number of 8 billion in November 2022 and is expected to grow further up to 9.7 billion in 2050 (United Nations, Department of Economic and Social Affairs, Population Division (2019) World Population Prosects 2019: highlights, United Nations, New York). Along with the increase in population, natural resources are increasingly consumed and become limited. One of the most fundamental challenges is to feed the world's growing population in sufficient quantities, rendering global food security as an important topic of global impact (Le Mouel et al., 2017, European Review of Agricultural Economics, 44 (4)). This particularly concerns the global demand for products derived from animals, including meat products, as livestock farming alone takes up more than 66% of the global agricultural land (Statista Research Department, 03.01.2024). Considering the low potential for further improvements of livestock faming, and the limited available land for agriculture, there is a need for alternative ways of producing products derived from animals. This is fired by growing attention of animal ethics but also the environmental risks associated with conventional livestock farming, especially greenhouse gas emission by the animals and decrease in biological diversity due to monoculture and use of pesticides for the production of feed for them. To fulfill the current and future demands for products derived from animals, scientists are increasingly looking into producing animal derived products in the lab with a focus on producing meat synthetically (referred to as "artificial", "lab", "cultivated", "clean", "cultured" or "in vitro" meat).

The advancements in biotechnology, particularly in the field of tissue engineering opens new possibilities for producing products that resemble animal derived products, particularly by culturing animal-derived cells in bioreactors. While tissue engineering for medical purposes is gaining increasing interest and is developing quickly, there is still a lack of producing edible tissues, such as meat, at large scale and in an affordable manner. Production in large scale is also crucial to meet consumer demands and achieve widespread market accessibility. In addition, closely resembling the natural animal derived product, such as meat, is crucial for consumer acceptance. However, realistic texture in artificially grown animal products is challenging, due to the high complexity of the tissue, including its hierarchical, highly organized structure but also the combination of different cell types, including muscle and fat cells, as well as endothelial cells.

Currently, an increasing number of approaches focuses on artificially producing minced meat or patty forms of meat, as these are relatively easy to recapitulate. This resulted in the world's first-ever lab-grown beef burger in 2013 with associated costs of $330,000. Since then, the technology has dramatically improved and scaled-up, such that prices of 2022 dropped to around $9 per burger (Forbes, March 8, 2022, "Making Meat Affordable: Progress Since The $330,000 Lab-Grown Burger"). However, minced meat comprises loose and small pieces which is simple compared to the natural structure of animal muscle tissue, such that it can be replicated using conventional cell culture methods. For instance, WO 2022/086926 A1 reports culturing cells in a plate or microfluidic chip to facilitate formation of spheroids or organoids, followed by scale-up in suspension culture.

On the other hand, whole muscles or muscle cuts have complex, highly organized structures with organized muscle fibers, connective tissues and fat distribution, which are challenging to replicate artificially - let alone at large scale and at an economically acceptable price. At present different approaches are investigated in the field, such as scaffolds of natural or synthetic materials upon which cells are seeded and cultured. Scaffolds should ideally mimic the surrounding the cells natively encounter, i.e. the so-called extracellular matrix (also referred to as "ECM"). Natively in the tissues, the cells create their surrounding throughout development and differentiation such that the ECM is cell-produced and constantly remodeled and adapted. Thus, a synthetic scaffold should provide an environment allowing cells to attach but also have enough space to allow cell proliferation and tissue maturation. In addition, the scaffold should ideally degrade over time to be replaced by the ECM produced by the cells. Present exemplary scaffold-based approaches rely on decellularized tissues, such as decellularized plant material, three-dimensional (3D) hydrogel matrices or (nano-)fibrous matrices (see Campuzano et al., 2020, bioRxiv 2020.02.23.958686, WO 2022/038240). One main challenge in prefabricated scaffolds is to seed sufficient cell numbers, especially for larger macroscopic constructs required to reproduce muscle tissue or meat for human consumption. Also, precisely seeding the cells at (different) positions throughout the scaffold is very complicated for prefabricated scaffolds. In line, precise seeding of different cell types within a macroscopic, prefabricated scaffold, e.g. in order to reproduce marbling of meat, has not been possible with prefabricated scaffolds. A technique to address this challenge is 3D bioprinting, wherein the cells are mixed with a bioink and extruded to print structures. By using multiple cartridges, different cell types and bioinks may be used to form complex multiphasic structures, which are subsequently cultured in a bioreactor for tissue formation. For instance, in 2021 Kang et al. aimed at producing in vitro steak-like tissue by printing three types of bovine cell fibers (muscle, fat, vessel) and manually assembling these into a larger construct (see Kang et al., 2021, Nature Communications, 12:5059). While the cell seeding can be precisely tuned, such process required manual assembly, is comparably inefficient and slow, and challenging to scale up. Moreover, 3D bioprinted constructs as well as other hydrogel-based approaches oftentimes lack sufficient material transport characteristics, as these have dense material characteristics resulting in only diffusion-based transport of nutrients and oxygen to the cells. In addition, most hydrogel-based approaches, including the hydrogel-forming inks typically used for bioprinting, are fairly dense and homogeneous and thus, do not provide the microscopic space or porous structure that encapsulated cells need for growth and maturation into a dense organized tissue. Finally, most scaffold or bioprinting based approaches are time-consuming, require manual processes, are complex and very expensive for large scale production of artificial meat.

Hence, there is a need for improving the production of artificial meat, especially with the object to mimic the organized structure and texture of naturally grown meat. It is further desirable to provide a method for allowing cells to form tissues to resemble meat, such as muscle tissue, including providing a suitable environment for the cells to stay viable, proliferate and form tissue-like structures. It is further desirable to provide a method capable of being scaled up for producing large quantities while keeping production costs low to achieve industry and consumer acceptance.

### SUMMARY OF THE INVENTION

The present invention addresses the above-described needs by providing a technology by which artificial meat production is improved. The present invention provides inter alia a method for producing a cell-containing fiber particularly suitable for producing artificial meat. The herein disclosed cell-containing fiber is produced by providing a microcarrier and cells capable of interacting with the microcarrier, as well as a composition comprising a compound capable of crosslinking or solidifying. These undergo spinning to generate a fiber, which is at least partly crosslinked or solidified. The combination of microcarrier and cells capable of interacting therewith advantageously allow for providing the cells a suitable (micro-)environment, e.g. by attaching to the microcarrier and/or receiving signals such as growth factors from the microcarrier. Furthermore, the cells can be upfront cultured with the microcarrier using established cell culture methods and be delivered at freely adjustable cell density. Thereby, existing large-scale production of microcarrier-based cell culture is advantageously utilized for producing the fiber but also artificial meat at high efficiency. At the same time, due to the spinning process, space between the microcarriers within the fiber is provided for the cells to grow into, such that the cells can proliferate and/or differentiate and form tissue within the fiber. On the other side, by being (at least initially) confined within the fiber, the cells are not washed out or released out of the fiber, providing an optimal environment forthe cells to grow, and form tissue-like structures. Moreover, the present invention allows for precisely delivering or seeding the cells in the fiber, allowing for a desired seeding density of cells in the fiber, as well as for larger constructs for producing artificial meat. In addition, different cell types may be seeded in the same or different fibers, which enables resembling the macroscopic heterogeneity, e.g. on whole cut meat/muscle tissue, such as (bovine) meat/muscle. The microcarrier additionally provides adequate support for the fiber structure, which is particularly advantageous for partially solidified or crosslinked fibers, such as core-shell hollow fibers, wherein the microcarrier is within the fiber core. Moreover, by utilizing the spinning-based approach, the fiber can be produced efficiently at large scale and in an inexpensive manner. The spinning-based approach further allows for straightforward automation. The fiber can be used to create a structure that is edible, mimics the texture, structure, and mouthfeel of real meat, and supports the growth of myocytes and other cell types, forming structures resembling natural muscles.

According to a first aspect, a method for producing a cell-containing fiber is provided, comprising:
(a) providing
   - a microcarrier,
   - cells capable of interacting with the microcarrier, and
   - a composition comprising a compound capable of crosslinking or solidifying;
(b) spinning the microcarrier, the cells and the composition to generate a fiber; and
(c) crosslinking or solidifying at least a part of the fiber.

According to a second aspect, a cell-containing fiber, preferably being edible, is provided comprising a microcarrier with cells interacting therewith, wherein at least a part of the fiber is crosslinked or solidified. The cell-containing fiber according to the second aspect advantageously provides a fiber that is highly suitable for producing artificial meat, particularly for mimicking structured or textured meat. The disclosed advantages of the method according to the first aspect are embodied in the cell-containing fiber according to the second aspect.

According to a third aspect, a cell-containing fiber, preferably being edible, produced by the method according to the first aspect is provided, wherein the microcarrier and/or the crosslinked or solidified at least part of the fiber are dissolved or degraded. The cell-containing fiber according to the third aspect advantageously provides a fiber that is highly suitable for producing artificial meat, particularly for mimicking structured or textured meat, but also providing a fiber that comprises less or no material contributed by the microcarrier and/or the crosslinked or solidified at least part of the fiber. This allows for producing a fiber similar to natural tissue, e.g. such as a muscle fiber, containing essentially only the cells and the produced extracellular matrix.

According to a fourth aspect, an edible, cell-containing fiber, preferably wherein the fiber is a core-shell hollow fiber, is provided for use as artificial meat.

According to a fifth aspect, a spinning system for producing a cell-containing core-shell hollow fiber (4) is provided, wherein the fiber comprises a microcarrier and cells, preferably a microcarrier with cells interacting therewith, the system comprising:
(i) a first container (1) for holding a microcarrier;
(ii) a second container (2) for holding a composition comprising a compound capable of crosslinking or solidifying;
(iii) a core-shell nozzle (3) for extruding the microcarrier through the core nozzle and extruding the composition through the shell nozzle; and
(iv) a collector for collecting the cell-containing core-shell hollow fiber;
wherein the first container (1) further is for holding the cells and/or the system comprises a further container for holding the cells.

The spinning system according to the fifth aspect advantageously allows producing a cell-containing core-shell hollow fiber suitable for producing artificial meat, particularly for mimicking structured or textured meat. Furthermore, utilizing the spinning system achieves producing the cell-containing core-shell hollow fiber efficiently at large scale and in an inexpensive manner, and allows automation.

According to a sixth aspect, a method for producing artificial meat is provided, comprising:
(I) producing one or more edible, cell-containing fiber(s), preferably wherein the fiber(s) are core-shell hollow fiber(s) and/or wherein the fiber(s) are fiber(s) according to the second or third aspect of the invention, preferably produced by a method according to the first aspect of the invention; and
(II) collecting and/or assembling the one or more fiber(s) for forming a three-dimensional construct.

The method for producing artificial meat according to the sixth aspect of the present invention advantageously allows for producing artificial meat, preferably meat having a texture or structure, e.g. whole meat/muscle or whole cut meat/muscle. In particular, by producing one or more edible, cell-containing fiber(s), as disclosed herein and subsequent collecting and/or assembling the one or more fiber(s) the formed three-dimensional construct can resemble meat artificially, especially structured meat. In addition, a plurality of edible, cell-containing fibers may be provided, which can resemble the marbling, which is referred to the intramuscular fat distribution within meat. Producing these at large mass scales, is advantageous in tissue-engineered meat production for meeting consumer demand and widespread market accessibility, cost efficiency, and potential environmental benefits.

According to a seventh aspect, artificial meat is provided comprising assembled and/or collected one or more edible, cell-containing fiber(s), preferably wherein the fiber(s) are core-shell hollow fiber(s) and/or wherein the fiber(s) are fiber(s) according to the second or third aspect of the invention, preferably produced by a method according to the first aspect of the invention.

Further aspects of the invention are disclosed below. Other objects, features, advantages, and aspects of the present application will become apparent to those skilled in the art from the following description and appended claims. It should be understood, however, that the following description, appended claims, and specific examples, while indicating preferred embodiments of the application, are given by way of illustration only.

### BRIEF DESCRIPTION OF THE FIGURES

Some exemplary embodiments of the present disclosure will now be described with reference to the accompanying drawings.

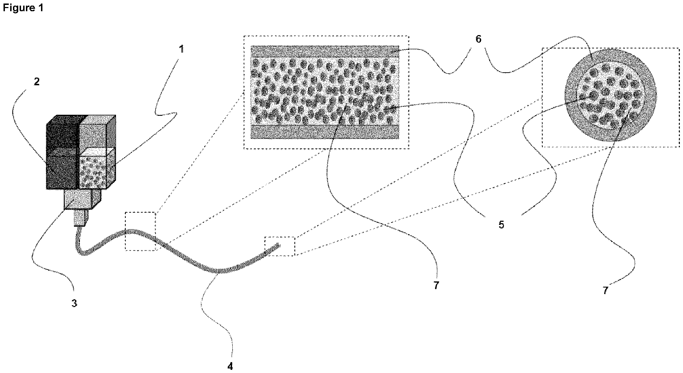

- Fig. 1: shows a schematic, exemplary representation of a spinning process and the formed composite hollow fibers of the present disclosure. Shown are microcarriers covered with cells distributed in a liquid (e.g. carrying medium) provided in a first container (1) and a composition comprising a compound capable of crosslinking or solidifying provided in a second container (2). By extruding these through a core-shell nozzle (3) the cell-containing, core-shell hollow fibers (4) can be produced, wherein the microcarriers and cells are distributed within the core (5) and the crosslinking or solidifying compound in the shell (6). Between the microcarrier voids (7) are present to form space for the cells to differentiate and/or proliferate for forming tissue.

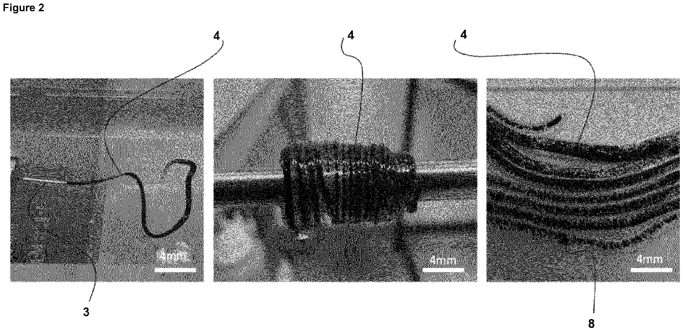

- Fig. 2: shows a custom-designed spinning setup, wherein the composition (here alginate solution) was extruded through a core-shell nozzle for producing core-shell hollow fibers (3). The nozzle had a core diameter of 300 µm and a shell diameter of 1 mm. The core-shell hollow fibers (4) were extruded into a coagulation bath (here containing dissolved CaCl2) for crosslinking at least part of the fiber, i.e. the fiber shell (see left picture). The fibers are then collected using a drum (10) which can be rotated for collecting and optionally stretching the fiber (see picture in the middle). The fibers can be collected with essential parallel orientation (see right picture). The core was filled with a bore solution comprising microcarriers (MCs, 8) suspended in liquid (here DMEM cell cultured medium).
- Fig. 3: shows a produced core-shell hollow fiber comprising MCs at day 0 right after spinning (left side) and after 10 days in culture (right side). The core of the fiber (5) contains the MCs and cells interacting therewith, whereas shell (6) comprises the crosslinked alginate. It can also be seen that after culture, the cells grow on top and in the space between the MCs (9). They also fill up larger empty spaces within the core and form linear muscle tissue (11) parallel to the long axis of the core-shell hollow fiber.
- Fig. 4: shows an exemplary fluorescence microscopy image of the core-shell hollow fiber after culturing C2C12 cells attached to MCs within the fibers. Scale bar is 500 µm.
- Fig. 5: shows an exemplary fluorescence microscopy image of the core-shell hollow fiber after culturing C2C12 cells attached to MCs within the fibers. Scale bar is 500 µm.
- Fig. 6: shows an exemplary fluorescence microscopy image of the core-shell hollow fiber after culturing C2C12 cells attached to MCs within the fibers. Scale bar is 200 µm.
- Fig. 7: shows an exemplary fluorescence microscopy image of the core-shell hollow fiber after culturing C2C12 cells attached to MCs within the fibers. Scale bar is 200 µm.
- Fig. 8: shows an exemplary fluorescence microscopy image of the core-shell hollow fiber after culturing C2C12 cells attached to MCs within the fibers. Scale bar is 200 µm.

### DETAILED DESCRIPTION

The following description serves to deepen the understanding of the present disclosure and shall be understood to complement and be read together with the description of exemplary embodiments of the present disclosure as provided in the above section of this description. It is to be understood that this invention is not limited to the particular embodiments, methodologies, protocols and reagents described herein as these may vary within the scope set by the claims. It is also to be understood that terminology used herein is for the purpose of describing particular embodiments only and is not intended to limit the scope of the present invention which is defined by the appended claims. Unless defined otherwise, all technical and scientific terms used herein have the same meanings as commonly understood by one of ordinary skill in the art.

In the following description, certain elements of the present invention will be described. These elements may be discussed with specific embodiments, however, it should be understood that they may be combined in any manner and in any number to create additional embodiments. The variously described examples, features and particular embodiments should not be construed to limit the present invention to only the explicitly described embodiments or to the explicitly described combination of features. This description should be understood to disclose and encompass embodiments which combine the explicitly described embodiments with any number of the disclosed and/or preferred elements. Furthermore, any permutations and combinations of all described elements in this application should be considered disclosed by this description unless the context indicates otherwise.

As explained in the summary of the invention, the different aspects and embodiments of the invention disclosed herein make important contributions to the art by providing improved methods for producing cell-containing fibers, preferably being edible, which are suitable for producing artificial meat.

### Method according to the first aspect of the invention

According to a first aspect, a method for producing a cell-containing fiber is provided, comprising:
(a) providing
   - a microcarrier,
   - cells capable of interacting with the microcarrier, and
   - a composition comprising a compound capable of crosslinking or solidifying;
(b) spinning the microcarrier, the cells and the composition to generate a fiber; and
(c) crosslinking or solidifying at least a part of the fiber.

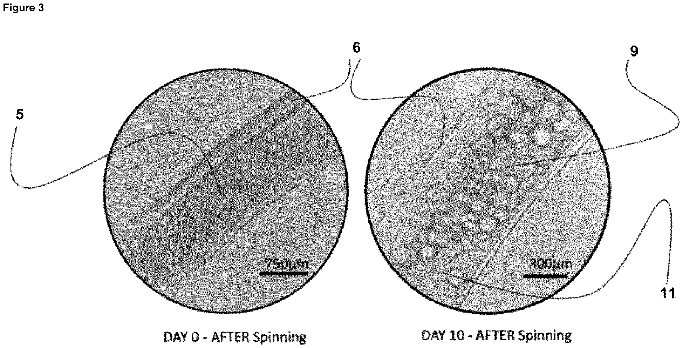

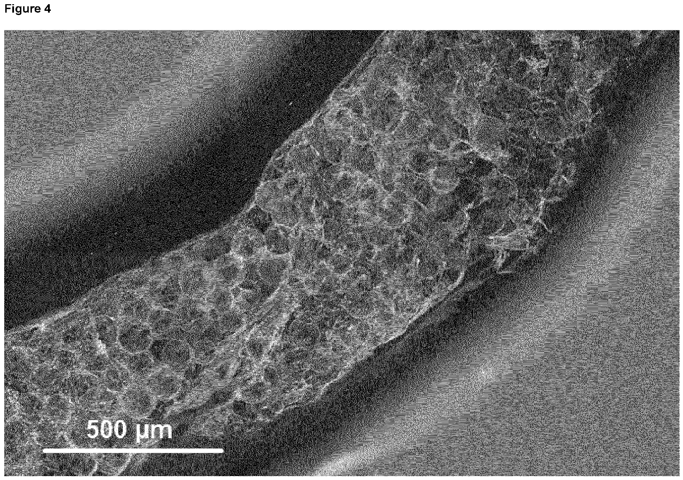

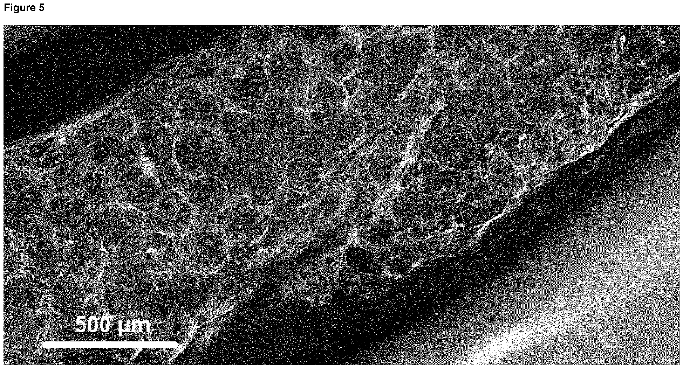

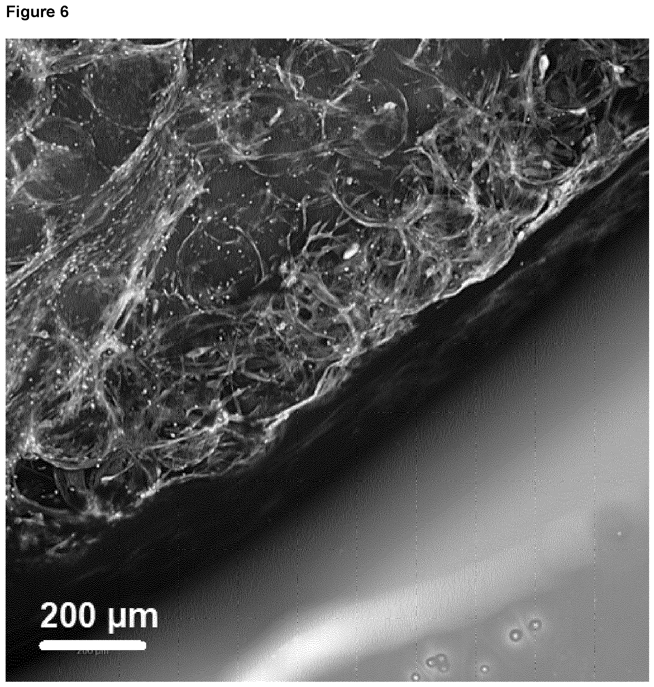

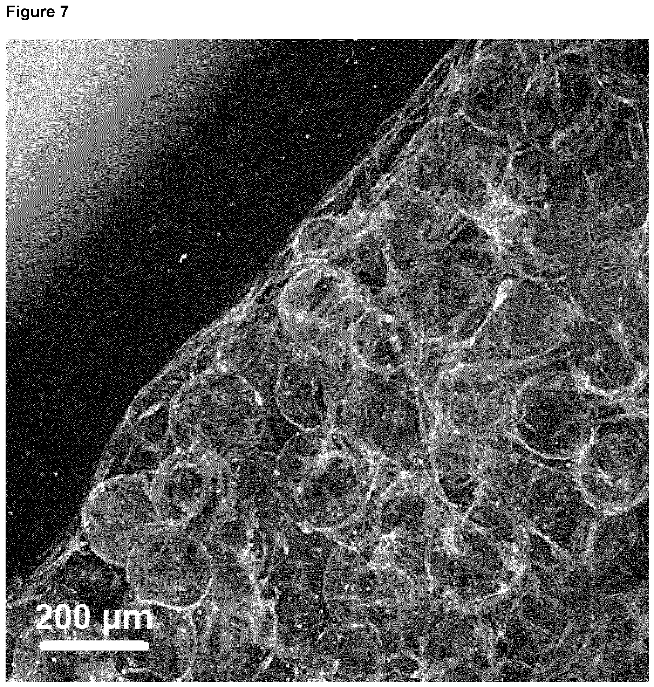

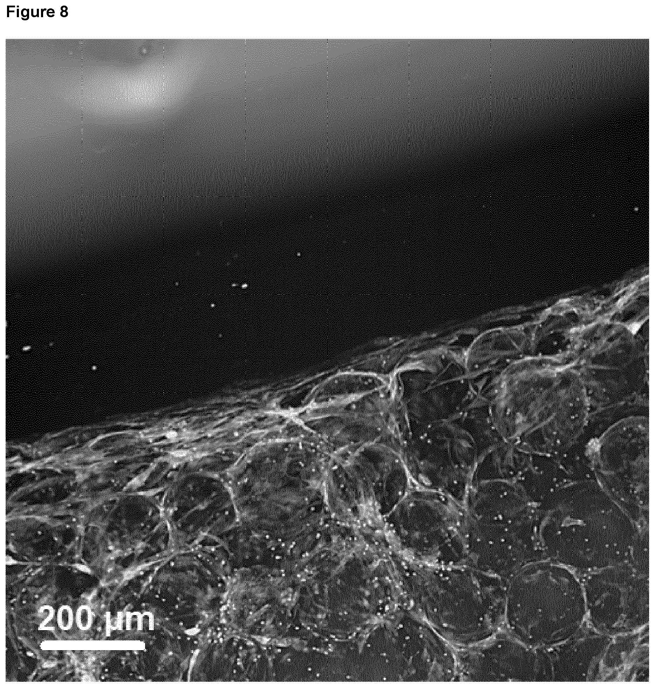

The method according to the present invention advantageously allows for producing a cell containing fiber suitable for producing artificial meat. In particular, by providing the microcarrier and cells capable of interacting with the microcarrier, the cells are provided an adequate surrounding to keep them viable and enable proliferation and/or differentiation, e.g. by allowing cells to attach thereto and/or by providing growth factors directly to the cells. Moreover, the microcarrier are a suitable carrier for the cells to be spun into the generated fiber. At the same time, the spinning process allows for space between the microcarriers in the fiber for the cells to grow into, such that the cells can proliferate and/or differentiate and form tissue within the fiber. On the other side, by being (at least initially) confined within the fiber, the cells are not washed out or released out of the fiber, providing an optimal environment for the cells to grow, and form tissue-like structures. Importantly, the present invention allows for precisely delivering or seeding the cells in the fiber, allowing for a desired seeding of the fiber, as well as larger constructs for producing artificial meat. By subsequent culture, it was observed that cells can grow further in the empty space of the fiber, particularly the core-shell hollow fiber (see Examples section). This enables populating the whole empty space of the fiber with cells (see **Fig. 3**). Moreover, the cells grew parallel to the long axis direction of the fibers (see **Figs. 4-8**), indicating that the cells produce an own aligned ECM similar to native muscle fibers, highlighting the suitability for artificial meat application. Furthermore, the cells within the fiber can be adequately supported by nutrients and oxygen, indicating excellent mass transport. In addition, different cell types may be seeded in the same or different fibers, which enables resembling the macroscopic heterogeneity, e.g. on whole cut meat/muscle tissue, such as (bovine) meat/muscle. The microcarrier additionally provides adequate support for the fiber structure, which is particularly advantageous for partially solidified or crosslinked fibers, such as core-shell hollow fibers, wherein the microcarrier is within the fiber core. Finally, by utilizing the spinning-based approach, the fiber can be produced efficiently at large scale and in an inexpensive manner, as well as automation is allowed.

The term "fiber" is used herein in line with the common understanding of these terms in the field of taxonomy and may interchangeable be used with the term "filament". A "fiber" according to the present disclosure is an elongated object with high aspect ratios, particularly aspect ratios of more than 2, preferably more than 5 or 10, or even more than 20, more than 30, more than 40 or more than 50. A fiber shall not be limited in its internal structure, such that a fiber can be single or multiphasic, including core-shell or other types of phase assembly. According to preferred embodiments, the fiber is monophasic or a core-shell hollow fiber, more preferably a core-shell hollow fiber. The fiber may also have different cross-sectional shapes, including circular, ellipsoidal, triangular, square, or rectangular. Preferably the cross-sectional shape is essentially or fully isometric. Preferably the fiber cross-sectional shape is ellipsoidal, more preferably circular, or essentially circular. As the fiber disclosed herein is "cell-containing", it is clear to the person skilled in the art that such a fiber has a sufficient size or diameter to comprise the cells (as well as the microcarrier), such as eukaryotic cells, preferably animal cells.

"Cell-containing" according to the present disclosure refers to the cells being present inside the fiber. Specifically, the cells may be in the fiber core, shell and/or at the very edge. It is not excluded that the cells are also present on the surface of the fiber or in any surrounding environment, such as surrounding fluid. The skilled person will understand that a fiber, wherein cells are purely based on the outer surface but not inside the fiber, are not considered cell-containing according to the present disclosure.

The fiber is most preferably edible. This is particularly advantageous for being suitable for being consumed, especially in form of artificial meat. Particular applications of the fiber can be in the food industry for producing cultured meat with texture and structures similar to conventional meat. The herein disclosed invention can enable the customization of meat alternatives with specific textures, flavors, and nutritional profiles, providing consumers with a wide range of options that closely resemble conventional meat products.

According to preferred embodiments, the fiber is suitable for medical purposes, including for transplantation, engineering tissues for drug testing and disease modeling, promoting wound healing, developing personalized medicine, and advancing cancer research. While the primary application of the fiber according to the present invention is for being consumed, e.g. in form of artificial meat, it is not excluded that the fiber is also being used as a medical product, e.g. by forming muscle tissue suitable for being implanted for medical purpose. While some aspects such as a low price and very high efficiency are less relevant for medical purposes compared to food, various advantages such as the close replication of muscle strands are equally or even more important for medical applications. Hence, the cell-containing fiber described herein is well suited for being applied for medical purposes.

### Step (a)

Step (a) according to the method of the first aspect comprises providing
- a microcarrier,
- cells capable of interacting with the microcarrier, and
- a composition comprising a compound capable of crosslinking or solidifying.

The microcarrier, cells and composition can be provided in any order or can be provided in a combined manner. According to preferred embodiments, the microcarrier and the cells in step (a) are provided as one solution or dispersion, wherein at least a part of the cells is attached to the microcarrier. This has the advantage, that the cells can already interact with the microcarrier in the solution or dispersion. For instance, the cells may be attached to the microcarrier when being provided in step (a) according to the present disclosure. According to one embodiment, the cells may be cultured before step (a) in a culture together with the microcarrier, e.g. in a suspension culture. This has the advantage that industrially well-established methods known by the skilled person can be utilized upfront to produce the cells attached to the microcarrier in adequate concentrations. Some cells may in addition be present which are not attached to the microcarrier. It may also be within the scope of the present disclosure that some cells are provided that are capable of interacting with, particularly attaching to, the microcarrier, whereas other cells that are provided do not interact or do not attach to the microcarrier.

According to preferred embodiments, the microcarrier and the cells in step (a) are provided as separate solutions and/or dispersions. Providing the microcarrier and cell separately has the advantage that the skilled person can flexibly decide on microcarrier and cell before spinning step (b) without mixing both. When being spun, the cells may advantageously interact with the microcarrier, such that the cells remain viable and proliferate. Preferably the cells may interact with the microcarrier in the produced fiber such that the cells attach to the microcarrier. Providing both separately also has the advantage that the cells do not start interacting with the microcarrier prior to being contacted, particularly in spinning step (b).

### Cells

The cells in provided in step (a) may be any cell type suitable for being delivered or seeded into the fiber. Hence, the skilled person understands that the cell type shall not be limited to a particular type of cells. Nevertheless, depending on the application, e.g. being edible or suitable for medical application, the skilled person will understand that certain cells may be more suitable to be provided in step (a). In general, the cells provided in step (a) are capable of interacting with the microcarrier. "Capable of interacting" according to the present disclosure shall encompass any type of interaction that a cell can have with the microcarrier. According to preferred embodiments, the cells are capable of interacting with the microcarrier by being capable of (i) attaching to the microcarrier and/or (ii) sensing chemical or biochemical cues originating from the microcarrier. In particular embodiments, the cells may be interacting with the microcarrier by attaching, e.g. adhering, to the microcarrier. In particular embodiments, the cells may interact with the microcarrier, by sensing or incorporating or interacting with molecules or moieties provided or presented by the microcarrier, e.g. wherein the microcarrier contains growth factors or other biological cues, sensed or incorporated by the cells. In such embodiments, the microcarrier may be used as a delivery vehicle for activating, differentiating, reprogramming, or performing other processes on the cells. Loading of biochemical molecules or cues onto/into microcarriers is well known by the skilled person and shall not be limiting in any manner within the present disclosure. In particular embodiments, the cells may interact with the microcarrier by degrading at least a part of the microcarrier, e.g. by enzymatic degradation. In particular embodiments, capable of interacting comprises cells attaching to the microcarrier and degrading the microcarrier throughout any sequential step, e.g. culturing step.

According to preferred embodiments, the cells are eukaryotic cells, preferably animal cells. Such cells are particularly suitable when fibers are produced that are used for animal derived products, especially animal derived meat. According to some embodiments, the cells are one or more cell types found in animal tissues, particularly native muscle tissue. According to preferred embodiments, the cells are one or more cell types found in meat, preferably one or more cell types found in bovine meat, galline meat, chicken meat, bird meat, goat meat, deer meat, rabbit meat, kangaroo meat, porcine meat, fish meat and/or ovine meat. According to preferred embodiments, the cells are one or more cell types found in an animal, preferably one or more cell types isolated or derived from bovine cells, galline cells, chicken cells, bird cells, goat cells, deer cells, rabbit cells, kangaroo cells, porcine cells, fish cells and/or ovine cells. The skilled person will recognize that the present invention shall not be limited to any particular type of meat and their respective cell types. For instance, the cells may also be one or more cell types found in lion meat, tiger meat, dinosaur meat, mammoth meat, or other less conventional types of meat.

"Meat" according to the present disclosure refers to flesh of an animal used for food. The present disclosure allows particularly for producing meat artificially/synthetically that comprises texture or structure, such as (whole) muscle (tissue), or muscle cut. The terms "meat" and "muscle" may be interchangeably used herein.

According to preferred embodiments, the cells are selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells. Such cell types are cells which are particularly suitable for being used in production of muscle tissue, such as meat tissue. According to particularly preferred embodiments, the cells comprise at least a myocyte and/or a muscle satellite cell, optionally further comprising adipocytes, mesenchymal stem/stromal cells, fibro/adipogenic progenitor cells, endothelial cells, and/or pluripotent stem cells. It may be advantageous to also combine different types of cells either within one fiber or by producing multiple fibers wherein the fibers can have different cell types. For instance, one fiber may comprise satellite cells or myocytes, another fiber may comprise adipocytes or fibro/adipogenic progenitor cells. A further fiber may comprise endothelial cells to include fibers suitable for vascularization. A further fiber may comprise progenitor or pluripotent stem cells, which can be differentiated at some point, e.g. during later cultivation. As a result of providing some fibers comprising different cell types, it is possible to mimic the marbling, which is referred to the intramuscular fat distribution within meat. Producing these at large mass scales, is advantageous in tissue-engineered meat production for meeting consumer demand and widespread market accessibility, cost efficiency, and potential environmental benefits.

The cells can be alive, apoptotic and/or dead. It may be particularly advantageous to provide alive cells, in order to allow culturing the cells contained in the fibers (see e.g. Examples below). However, some cells may be apoptotic or dead. In some embodiments, most cells can be apoptotic or dead when provided in step (a), which can be suitable when the fibers are after production not further cultured but directly made available for consumption.

The cells may be differentiated cells, non-differentiated cells, pluripotent cells, multipotent cells, reprogrammed cells, or a combination thereof. In some embodiments, the cells are already differentiated cells such that the cells remain one cell type and proliferate in the fiber for tissue formation. In some embodiments, the cells are pluripotent, multipotent or reprogrammed cells such that the cells remain in that state or can be further differentiated in the fiber for tissue formation. In some embodiments, combinations of differentiated and pluripotent, multipotent or reprogrammed cells may be provided and be present in one or multiple fibers. This can be adapted according to the needs, particularly for closely resembling the structure of the tissue to be produced. For instance, in meat tissue it may be advantageous to provide differentiated muscle cells or satellite cells but also include some progenitor cell types, similar to native tissues.

### Microcarrier

The microcarrier is provided in step (a). It advantageously is capable of interacting with the cells, particularly by providing a support matrix. The term "microcarrier" is used herein in line with the common understanding of these terms in the field of taxonomy. A "microcarrier" according to the present disclosure may be any type irrespective of shape or chemical nature. The microcarrier may be referred to herein as "microcarriers", "MC", MCs", or "structural element". The term "microcarrier" herein encompasses both the singular form, i.e. a single microcarrier, and the plural form, i.e. multiple microcarriers. Microcarriers are commonly known as a support matrix that can allow the growth of adherent cells in bioreactors. The typically three-dimensional (3D) support structure allows to carry several hundred cells, and thereby increases the expansion capacity over conventional two-dimensional systems. Microcarriers are known for being a straightforward way to scale up cells to industrial scale, rendering these particularly suitable forthe present invention, especially for producing artificial meat at large scale. For the microcarrier according to the present disclosure it may be particularly advantageous to provide microcarriers that are edible. Such edible microcarriers are known in the field and the skilled person is readily aware (see e.g. U.S. 9,752,122 B2, WO 2023/104768 A1). Note that such edible microcarriers alone are unable to produce the desired mouthfeel or texture akin to meat tissue and are unable to achieve uniformly structured cell densities comparable to actual tissue at large scale production. But utilizing (edible) microcarriers as in the present disclosure as support structure for the cells which particularly are confined in the fiber is advantageous, allowing precise control and easy upfront scale-up to achieve large cell numbers.

According to preferred embodiments, the microcarrier comprises a surface that is suitable forthe cells to attach thereto, preferably suitable for anchorage-dependent cells. According to preferred embodiments, the microcarrier comprises a cell-adhesive surface. Such surfaces are particularly advantageous for cells requiring attachment, e.g. satellite cells. Thereby, a suitable support matrix is provided allowing the cells to attach, remain viable and proliferate. These may also provide adequate support for stem cells in order to provide mechanical or biochemical cues for stem cell differentiation. On the other side, when providing cells that do not require adhesion, e.g. adipocytes, the surface does not have to be suitable for adhering to (such) cells. In such cases, the cells may interact with the microcarrier by sensing chemical or biochemical cues originating from the microcarrier.

Preferably, the surface of the microcarrier is charged, such as positively or negatively charged. The microcarrier may also be amphiphilic. The charged surface facilitates interaction with the cells, preferably by enabling attachment of the cells. Suitable methods and techniques to create charged surfaces of microcarriers are readily known by the person skilled in the art. According to a preferred embodiment, the microcarrier has moderate hydrophilicity, in particular having a water contact angle of less than 90°. The water angle may be selected from the range of 0° to less than 90°, 5° to 75°, or 10° to 50°, preferably 20 to 40°. Hydrophilic materials are more suitable for interacting with the cells particularly by being better wettable in water but also by enabling hydrophilic interactions with the cells. According to preferred embodiments, the microcarrier surface is rough, porous or structured, or any combination of these. Such surfaces provide a greater area and thus more area for interacting with the cells, e.g. by providing more attachment points and/or more surface upon which biochemical interaction can take place.

The surface may preferably comprise a cell adhesive moiety. Such moiety may be recognized by one or more cellular integrins. Suitable adhesive moieties are readily known by the person skilled in the art. These may be selected from one or more of laminin, fibronectin, collagen, gelatin, vitronectin or fragments or peptides thereof, including RGD or IKVAV or a peptide sequence comprising these. In general, such moieties are advantageous to support attachment of the cells to the microcarrier and support their viability and/or growth. These can also induce signaling, e.g. by activating pathways, such as support differentiation and/or maturation of cells into tissue.

According to preferred embodiments, the microcarrier is partially or fully covered by cells attached thereto. The degree of coverage by the cells shall not be limiting, as it can be flexibly decided how high the degree of cell coverage may be. For instance, it may be advantageous to start with a high cell coverage in order to deliver more cells to the fiber. It may also be advantageous to start with a lower cell coverage, requiring less upfront scale-up time and/or enabling more cell proliferation and possibly tissue maturation within the fiber.

According to preferred embodiments, step (a) further comprises providing a microcarrier without cells attached thereto. In a particular embodiment, the microcarriers and cells may be cultured upfront, such that the cells attach to the microcarrier. In such embodiment, the cells may be scaled up in such manner. When providing the microcarrier with cells attached thereto, further microcarriers may be added or co-provided for the spinning process in step (b) to generate the fiber. Such embodiments can be particularly advantageous to reduce the number of cells required for being included in the fiber, increasing overall process efficiency. At the same time, the available microcarriers without cells attached thereto are available for the cells to interact therewith. For instance, the cells may have further attachment opportunities and/or possibilities to biochemically interact, e.g. by receiving biochemical signals or cues. In some embodiments, microcarriers without cells attached thereto may be provided that serve as attachment points and/or other microcarriers without cells attached thereto may be provided that deliver biochemical signals to the cells, e.g. growth factors. In preferred embodiments, the volume or mass ratio of provided microcarrier with cells attached thereto to provided microcarrier without cells attached thereto is between 10:1 to 1:1000, preferably 2:1 to 1:100, more preferably 1:1 to 1:100 or 1:1 to 1:50. Such ratios have been found suitable for delivering cells with high efficiency. This can be particularly advantageous to provide further microcarriers for the cells to interact with but at the same time provide less cells.

According to preferred embodiments, the microcarrier material is edible and/or suitable for medical purposes, particularly selected from one or more of:
(i) monosaccharides, oligosaccharides or polysaccharides, such as alginate, cellulose, chitosan, hyaluronan, agar, inulin, pectin, methyl cellulose, dextran, tapioca, xanthan gum, guar gum, tara gum, bean gum, starch, gum Arabic, carboxymethyl amylose, carboxymethyl chitosan, chrondoitin sulfate, dermatan sulfate, heparin, heparin sulfate, natural mixes, including corn syrup, honey, maple syrup, or glucose syrup, or a combination thereof, preferably polysaccharides, more preferably, alginate, pectin, methyl cellulose, chitosan, dextran, or a combination thereof;
(ii) proteins, preferably gelatin, collagen, fibrinogen, fibrin, fibronectin, elastin, laminin, soy protein, zein protein, pea protein, canola protein, carob protein, cardosine A, wheat protein, albumin, casein protein, potato protein, suar protein, gluten, legume protein, corn protein, sorghum protein, animal protein, animal protein isolate, beef protein isolate, whey protein;
(iii) sugar alcohols;
(iv) protein hydrolysates; or
(v) a combination of any of (i) to (iv).

Such materials have been found useful for producing cell-containing fibers according to the present disclosure. In particular, such materials are edible and/or suitable for medical applications.

Microcarriers can include biochemical cues to be delivered to presented to the cells. For instance, it may be desirable to include growth factors and/or differentiation factors to the cells. This can enable interaction with the cells (in addition or next to cells attaching to the microcarriers).

The microcarrier can have various shapes, such that the shape shall not be limiting in scope of the present invention. In preferred embodiments, the microcarrier has an ellipsoidal shape, a spherical shape, a cylindrical shape, a rod-like shape, a cuboid shape, a cone shape, an irregular or random shape, an isotropic shape, or an anisotropic shape, preferably, an ellipsoidal or rod-like shape. Such shapes have proven to be suitable for cells interacting therewith, e.g. by providing a surface shapes suitable for attaching with cells.

According to preferred embodiments, the microcarrier is porous, non-porous or hollow. In particular, porous or hollow microcarriers may provide more surface area for the cells to interact with. On the other side, non-porous or hollow microcarriers may provide a more suitable matrix to deliver biochemical signals over a longer time, as the surface to volume ratio limits diffusion.

According to preferred embodiments, the microcarrier is degradable or dissolvable, such as enzymatically degradable and/or degradable or dissolvable by a chelating agent. By being degradable or dissolvable, the microcarrier can be removed after fiber production, e.g. by the cells and/or external triggers. For instance, the fiber may after production be cultured and the cells may degrade the microcarriers enzymatically or biochemically. In other embodiments or in addition thereto, one may add a compound, such as a chelating agent and/or enzyme, in order to degrade to dissolve the microcarrier. As a result, the microcarrier is at least partially, preferably essentially completely, removed when being applied, e.g. for artificial meat, or for medical purposes, such as engineered tissue for implantation. This can be advantageous in order to produce a cell-containing fiber without material contributed by the microcarrier, preferably a fiber similar to natural tissue, e.g. such as a muscle fiber, containing only the cells and a produced surrounding extracellular matrix (ECM).

The microcarrier preferably has a size selected from 10 µm to 1,000 µm, 15 µm to 900 µm, 20 µm to 800 µm, 25 µm to 750 µm, 30 µm to 700 µm, 35 µm to 650 µm, 40 µm to 600 µm, 45 µm to 550 µm, preferably 50 µm to 500 µm, 60 µm to 450 µm, 70 µm to 450 µm, 80 µm to 400 µm, 90 µm to 350 µm, more preferably 100 µm to 300 µm. Such sizes have been found useful for being provided in step (a) and spun as a fiber. Moreover, such sizes are known in the field for microcarriers, such that these are readily available to the person skilled in the art.

The microcarrier may preferably be dispersed in a liquid, since it is advantageous to disperse the microcarrier in a liquid in order to spin the fiber, especially in a wet-spinning approach. Preferably the liquid is non-crosslinked, partially crosslinked or crosslinked, more preferably the liquid being non-crosslinked or partially crosslinked. The liquid can initially be non-crosslinked or physically crosslinked and during the spinning process extruded. When delivered in the fiber, the liquid may remain non-crosslinked or reform the physical crosslinks or may undergo crosslinking. It is particularly advantageous to provide a liquid that is provides sufficient space for the cells to growth and proliferate, which may be achieved by providing a non-crosslinked liquid or a liquid that is partially crosslinked or crosslinked but allows creation of space or voids for the cells, e.g. by phase separation or by thermal separation. It may also be possible to provide a liquid, wherein the microcarrier is dispersed and additionally particles that are later removed. In such a case, the liquid may be crosslinked or crosslinks after or during spinning. After producing the fiber, the additional particles may be removed, e.g. by leaching, such that space is created for the cells to grow. The liquid is in particular suitable for storing and/or culturing the cells at least for a short time, such as at least for 15 min, 30 min, 45 min or 60 min, preferably more than 60 min. This is particularly advantageous, when the microcarrier dispersed in the liquid and the cells are provided together in step (a), as the cells remain viable in the dispersion or solution during the production of the fiber. The liquid may comprise a cell culture medium, preferably it essentially consists of a cell culture medium, more preferably the liquid is a cell culture medium. A cell culture medium is particularly suitable when providing the cells together with the microcarrier for the production of the fiber to keep the cells viable. The liquid may be thermo-responsive, physically or chemically crosslinked, or ionically crosslinked. In such embodiments but also in other embodiments, it may be particularly preferred that the liquid is capable of undergoing phase separation, is degradable or non-degradable; and/or is dissolvable and/or degradable, preferably using a chelating agent and/or an enzyme, more preferably a combination of a chelating agent and an enzyme. By allowing some kind of removal of the liquid space can be created for the cells to grow and proliferate and/or provide a fiber from which the liquid can be removed, e.g. in order to produce a fiber similar to natural tissue, e.g. such as a muscle fiber, comprising the cells and synthesized ECM.

### Compound capable of crosslinking or solidifying

A composition comprising a compound capable of crosslinking or solidifying is provided in step (a) according to the method of the first aspect of the present invention. The composition is then spun together with the microcarrier and cells capable of interacting with the microcarrier to generate a fiber, and at least partially solidified or crosslinked. In particular, the at least partial solidifying or crosslinking is achieved by the composition comprising a compound capable of crosslinking or solidifying. This is important for the produced cell-containing fiber in order to create stability of the fiber and preferably provide some kind of confinement for the cells, such that the fiber remains cell-containing also after producing the fiber and does not lose its fiber structure. Also, such fiber structure is particularly suitable to mimic the natural tissue (fibers) and produce textures meat/muscle.

According to preferred embodiments, the compound capable of crosslinking or solidifying is capable of forming a solid matrix, preferably a solid polymeric matrix, optionally, wherein chemical crosslinks and/or physical crosslinks are formed. Such solid matrix, preferably a solid polymer matrix has been found to provide suitable for the cell-containing fiber, particularly for being used for producing artificial meat or being applied for medical purposes.

According to preferred embodiments, the compound is a monomer, prepolymer or polymer, preferably a polymer. Different chemical compound structures may be used in order to achieve crosslinking or solidification, wherein monomers or prepolymers may be crosslinked either with each other or by additionally providing a crosslinking reagent, e.g. during spinning or after spinning, such as a coagulation bath for generating ionic crosslinks (see Examples below, e.g. **Fig. 1**). A polymer may be used which is thermo-responsive or shear-thinning, such that it can be extruded in the spinning process but allows solidification or crosslinking afterwards.

The compound capable of crosslinking or solidifying can be physically or chemically crosslinked or can be ionically crosslinked. Such crosslinking allows for crosslinking at least part of the fiber, as is also demonstrated e.g. for ionic crosslinking in the Examples below (see e.g. **Figs. 1-8**). In some embodiments, the compound capable of crosslinking or solidifying is thermo-responsive allowing manipulating the solidification or crosslinking by a temperature switch, which can be advantageous in order to remove the compound, e.g. by a temperature shift. In some embodiments, the compound capable of crosslinking or solidifying can undergo phase separation. This can be advantageous in order to create further voids which allow cells to infiltrate and provide further space for growth and tissue formation. Various techniques and processes are known for crosslinking or solidifying using different types of chemistries or biochemistries (see e.g. Kyburz and Anseth, Ann. Biomed. Eng., 2015, Vol. 43(3), pp. 489-500; Rice et al., Advanced Healthcare Materials, Vol. 2(1), pp. 57-71). These are well-known by the person skilled in the art and shall not limit the scope of the present invention. Nevertheless, some chemistries or biochemistries may be more suitable, e.g. which allow for creating solidification or crosslinking of at least part of the fiber and aims at recapitulate some aspects of the native environment of the cells contained in the fiber. For instance, it may be desirable to provide mechanical, physical, and/or biochemical (micro-)environments for the cells that facilitate the cells to remain viable, proliferate and grow into tissues and/or differentiate into the desired cell type(s). For instance, it may also be desirable to provide mechanical, physical, and/or biochemical (micro-)environment(s) that closely match the artificial meat to be produced, such that the sensory feeling and taste is similar to livestock produced meat.

In some embodiments, the compound capable of crosslinking or solidifying is degradable or non-degradable, wherein being degradable is preferred in order to allow the compound capable of crosslinking or solidifying being removed by degradation. In some embodiments, the compound capable of crosslinking or solidifying is dissolvable and/or degradable, preferably using a chelating agent and/or an enzyme, more preferably a combination of a chelating agent and an enzyme. By being degradable or dissolvable, the compound can be removed after fiber production, e.g. by the cells and/or external triggers. For instance, the fiber may after production be cultured and the cells may degrade the compound enzymatically or biochemically. In other embodiments or in addition thereto, one may add a compound, such as a chelating agent, in order to degrade to dissolve the compound. As a result, the compound is at least partially, preferably essentially completely, removed when being applied, e.g. for artificial meat, or as medical material, such as engineered tissue for implantation. This can be advantageous in order to produce a cell-containing fiber without material contributed by the compound, preferably a fiber similar to natural tissue, e.g. such as a muscle fiber, containing only the cells and a produced surrounding extracellular matrix (ECM). In preferred embodiments, the compound capable of crosslinking or solidifying is biocompatible.

According to preferred embodiments, the compound is edible and/or suitable for medical purposes, particularly selected from one or more of
(i) monosaccharides, oligosaccharides or polysaccharides, such as alginate, cellulose, chitosan, hyaluronan, agar, inulin, pectin, methyl cellulose, dextran, tapioca, xanthan gum, guar gum, tara gum, bean gum, starch, gum Arabic, carboxymethyl amylose, carboxymethyl chitosan, chrondoitin sulfate, dermatan sulfate, heparin, heparin sulfate, natural mixes, including corn syrup, honey, maple syrup, or glucose syrup, or a combination thereof, preferably polysaccharides, more preferably, alginate, pectin, methyl cellulose, chitosan, dextran, or a combination thereof;
(ii) proteins, preferably gelatin, collagen, fibrinogen, fibrin, fibronectin, elastin, laminin, soy protein, zein protein, pea protein, canola protein, carob protein, cardosine A, wheat protein, albumin, casein protein, potato protein, suar protein, gluten, legume protein, corn protein, sorghum protein, animal protein, animal protein isolate, beef protein isolate, whey protein;
(iii) sugar alcohols;
(iv) protein hydrolysates; or
(v) a combination of any of (i) to (iv).

Such materials have been found useful for producing cell-containing fibers according to the present disclosure. In particular, such materials are edible and/or suitable for medical applications.

According to preferred embodiments, the composition provided in step (a) further comprises a liquid in which the compound is dissolved or dispersed. This is in particularly advantageous in order to provide the compound at a desired concentration in order to provide a solidification or crosslinking degree as desired. For instance, it may be desired to provide dilutions of less than 90wt% compound in the composition, preferably less than 70wt%, less than 60wt%, less than 50wt%, or less than 40wt%, e.g. 35wt%, 30wt%, 25wt%, 20wt%, 15wt%, 10wt%, 9wt%, 8wt%, 7wt%, 6wt%, 5wt%, 4wt%, 3wt%, 2wt%, or 1wt%. A suitable dilution is known by the person skilled in the art and can be flexibly decided on depending on the desired fiber and its characteristics. In preferred embodiments, the liquid is selected from water (including tap water, pure water, distilled water, deionized water, RO water, RO-EDI water, ultrapure water), cell culture medium, phosphate-buffered saline (PBS), physiological saline or the like, with water being particularly preferred. It is advantageous to use a liquid that is biocompatible, preferably being compatible or highly compatible with the cells, such that the cells are not significantly negatively affected. At the same time, the liquid needs to be compatible with the compound and allow the compound to be crosslinked or solidified. Thus, a water-based liquid may be desired, such as buffered water, pure water, or cell culture media.

### Step (b)

The method according to the first aspect of the present invention further comprises step (b) spinning the microcarrier, the cells and the composition to generate a fiber. Spinning is a manufacturing process for creating a fiber. It is well-known form the textile industry but also for tissue engineering purposes, e.g. as disclosed in Omidinia-Anarkoli et al., 2020, wherein a fiber platform was generated to study the behaviour of neurites (see Omidinia et al., 2020, Acta Biomaterialia, Vol. 113, pp. 350-359). "Spinning" according to the present disclosure shall refer to a process, wherein the microcarrier, the cells and the composition provided in step (a) are extruded through a spinneret or nozzle or needle or orifice to generate a fiber. Different types of spinning processes or methods are known in the art and are suitable to be used in frame of the present disclosure. Compared to the state of the art for producing artificial meat, fiber extrusion processes are faster, scalable for mass production, and involve simpler and more cost-effective equipment. The versatility of extrusion in handling various ingredients, its established technology with a proven track record, and its overall cost-effectiveness rendering it particular suitable for large-scale production of textured cultured meat. Additionally, this technology has the capability to streamline the manufacturing process, as a downstream step in any cultured minced meat facility. Spinning may in preferred embodiments not include bioprinting.

According to preferred embodiments, spinning step (b) is performed by a process selected from
(i) melt spinning, extrusion spinning or direct spinning; or
(ii) solution spinning, such as dry spinning, wet spinning, gel spinning or electrospinning; or
(iii) a combination of any process of (i) and/or (ii).

It is particularly preferred to use solution spinning, more preferably wet spinning. These spinning processes have been shown to be particularly suitable for being applied for cells, especially when these cells subsequently need to be cultured. This is also demonstrated in the Example below, wherein a wet spinning process has been used (see e.g. **Fig. 2**). Wet spinning is a flexible process because it allows for the adjustment of numerous parameters, and it is compatible with living cells. In this technique, a composition comprising a compound capable of solidifying or crosslinking, preferably dissolved with a liquid, e.g. a mixture of polymer and solvent, is spun into a nonsolvent, coagulation, or crosslinking bath, leading to at least partial crosslinking or solidification. The formation of a core-shell hollow fiber is possible using the wet spinning technique, e.g. by using a core-shell nozzle. In case, it is not planned to culture the cells after producing the fiber, also processes may be used that may cause cell death.

According to preferred embodiments, the fiber has a diameter selected from the range of 25 µm to 10,000 µm, 50 µm to 5,000 µm, 60 µm to 4,000 µm, 70 µm to 3,500 µm, 80 µm to 3,000 µm, 90 µm to 2,500 µm, preferably 100 µm to 2,000 µm, 120 µm to 1,800 µm, 140 µm to 1,600 µm, 150 µm to 1,400 µm, 180 µm to 1,200 µm, more preferably 200 µm to 1,000 µm or 500 µm to 1,000 µm. Such fiber diameters can be flexibly produced as demonstrated in the Examples (see e.g. **Figs. 2**, 4-8) and are suitable for being applied for artificial meat and/or medical applications.

According to preferred embodiments, the composition provided in step (a) is extrudable through a nozzle for spinning the fiber in step (b). This is particularly advantageous when using a nozzle-based approach during spinning step (b). According to preferred embodiments, composition provided in step (a) is liquid or becomes liquid upon spinning the fiber in step (b). This can be advantageous in order to facilitate spinning in step (b).

### Step (c)

The method according to the first aspect also comprises step (c) crosslinking or solidifying at least a part of the fiber. The crosslinking or solidification of at least part of the fiber allows for the produced fiber to remain structurally robust. Furthermore, by being (at least initially) confined within the fiber, the cells are not washed out or released out of the fiber, providing an optimal environment for the cells to grow, and form tissue-like structures. "Crosslinking" or "solidifying" according to the present disclosure may refer to an active or passive step, i.e. an external or internal trigger may lead to crosslinking or solidification or the material by itself may crosslink or solidify. The particular way of crosslinking or solidifying shall not be limiting in scope of the present invention, as long as the resulting fiber remains structurally intact after spinning and does not (directly) lead to release or wash out of the cells out of the fiber.

In general, method steps (b) and (c) may occur at the same time or sequentially. While it is not excluded that some degree of crosslinking or solidification may already have occurred prior to spinning step (b), at least some degree of crosslinking or solidification shall occur during or after spinning step (b) in order to crosslink or solidify at least part of the fiber in step (c).

Crosslinking or solidifying in general can be achieved by various techniques, processes or (bio-)chemistries according to the present disclosure, such that the particular mode of crosslinking or solidifying shall not be limiting. Some techniques, processes or (bio-)chemistries may, however, be more suitable, e.g. which allow for creating solidification or crosslinking of at least part of the fiber and aims at recapitulate some aspects of the native environment of the cells contained in the fiber. For instance, it may be desirable to provide mechanical, physical, and/or biochemical (micro-)environments for the cells that facilitate the cells to remain viable, proliferate and grow into tissues and/or differentiate into the desired cell type(s). For instance, it may also be desirable to provide mechanical, physical, and/or biochemical (micro-)environment(s) that closely match the artificial meat to be produced, such that the sensory feeling and taste is similar to livestock produced meat. Crosslinking or solidifying according to the present disclosure may be achieved by the compound capable of crosslinking or solidifying itself, e.g. after or during fiber spinning in step (b), the compound solidifies or crosslinks without any additional material or change of condition. For instance, the compound by solidify or crosslink over time, or may during extrusion in the spinning process liquify (e.g. shear-thinning materials) and solidify or crosslink, such that the spun fiber at least partially crosslinks or solidifies. In some embodiments, the compound capable of crosslinking or solidifying is combined with another compound or reagent, which achieves solidification or crosslinking together. For instance, the compound may be co-spun, e.g. by co-extrusion, such that the dual compounds are contacted during spinning or just shortly before. The solidification or crosslinking is then happening during and after spinning step (b). Examples are compounds reacting via click chemistry, e.g. thiol-ene chemistry, also referred to as thiol-Michael addition click reaction, or epoxy-amine reactions. Other suitable chemistries are known by the skilled person and can be ready applied. In some embodiments, the fiber containing the compound capable of crosslinking is subjected to a condition which facilitate at least partial crosslinking or solidification. For instance, the spun fiber may be subjected to certain environmental conditions (e.g. temperature or pressure shift), or irradiation (e.g. UV/VIS irradiation, NIR irradiation). In some embodiments, the fiber may be subjected to a fluid, e.g. a solution or dispersion containing a compound or reagent which reacts with the compound capable of solidifying or crosslinking, such that at least part of the fiber solidifies or crosslinks in step (c). For instance, the fiber may be subjected to a coagulation bath, e.g. containing calcium ions, which results in at least partial solidification or crosslinking of the fiber by a reaction of the compound capable of crosslinking with the coagulation bath, e.g. a reaction between calcium ions and alginate to form ionic crosslinks (see also Examples below, **Fig. 2**).

In preferred embodiments, the fiber is a polymeric fiber, preferably wherein crosslinking or solidifying at least a part of the fiber in step (c) comprises a crosslinked or solidified polymer matrix of at least a part of the fiber. Polymeric fibers may be particularly advantageous in scope of the present disclosure, as suitable polymers, prepolymers or monomers are available for the skilled person to crosslink or solidify by available chemical or biochemical reactions. Also, polymeric materials are widely applied in medicine and tissue engineering, e.g. in form of hydrogels, rendering such materials particularly suitable for the cell-containing fiber according to the present disclosure.

According to preferred embodiments, crosslinking or solidifying at least a part of the fiber in step (c) comprises subjecting the fiber to one or more of the following:
(i) a nonsolvent of the compound capable of crosslinking or solidifying;
(ii) a change in one or more conditions, preferably a change in temperature, humidity, shear forces, or pressure;
(iii) a crosslinking composition comprising one or more crosslinking reagents for crosslinking the compound capable of crosslinking or solidifying, preferably to form a solid polymer matrix, more preferably the crosslinking composition comprises ions for forming ionic crosslinks;
(iv) one or more enzyme(s), optionally including further co-factors or compounds required for the enzymatic reaction, or
(v) irradiation, preferably irradiation with light, such as NIR, visible or UV light.

It may also be possible to combine any of (i) to (v) depending on the reaction to be performed in order to crosslink or solidify at least part of the fiber. Subjecting the fibers to any of (i) to (v) is useful for crosslinking or solidifying the fiber, e.g. as demonstrated for the crosslinking composition in the Examples (particularly a coagulation bath for ionically crosslinking the compound capable of crosslinking or solidifying, see e.g. **Fig. 1**).

According to preferred embodiments, a further composition is provided capable of reacting with the compounds capable of crosslinking or solidifying, wherein the composition and the further composition are contacted prior to, during or after spinning step (b), preferably wherein during or after spinning step (b), and at least part of the fiber undergoes crosslinking or solidifying of step (c). Such an embodiment is advantageous in order to allow solidification or crosslinking without requirement of any additional compounds or reagents, but crosslinking occurs directly before, during and/or after spinning. Examples of such dual component reactions are readily known by the skilled person in the art. For instance, two monomers or prepolymers containing suitable moieties to react with each other, optionally by providing additional reaction triggers, may be contacted prior to or during spinning, e.g. by co-extruding these, react with each other just prior to, during, and/or after spinning step (c).

Crosslinking in step (c) according to the method of the first aspect leads to at least partial solidification or crosslinking of the fiber. In general, this covers but shall not be limited to crosslinking or solidifying
(i) the fiber shell, preferably not the fiber core; or
(ii) the complete fiber.

It is particularly advantageous according to the present invention to solidify or crosslink the fiber shell, but preferably not the fiber core. As a result, the cells and microcarrier remain in the fiber core in a (micro-)environment that may not be crosslinked or solidified and thus provide sufficient space for the cells to grow. At the same time, the cells remain within the fiber, i.e. the fiber continues to contain the cells, as the shell provides (at least initially) a barrier for the cells to lead to some kind of confinement (see Examples, e.g. **Figs. 3-8**). In other embodiments, the complete fiber is crosslinked or solidified in step (c). This can be advantageous in order to increase the barrier function of the fiber and trap the cells inside (see Examples below). On the other side, this can limit the space for the cells to grow, which is why in preferred embodiments, the fiber further contains voids for the cells to grow into. This may be achieved by different techniques, e.g. phase separation, temperature shift, material leaching etc. known in the art.

In general, either way (or other ways) of crosslinking (fiber shell but not core or complete fiber) is advantageous, as the produced fiber allows for controlling the cell density, type, cell (micro-)environment, etc. even in larger constructs, where the cell-containing fibers may be assembled into larger structures. Compared to techniques where cells are primarily seeded in a sequential step, such as after production of a (larger) scaffold structure, the control of the cell seeding is significantly improved. For instance, in constructs where cells are seeded sequentially in a 3D (macroscopic) construct, it is very challenging to seed the cells adequately throughout the whole construct, let alone allowing different cell types to be seeded in a controlled manner. In addition, by providing the microcarrier of the present disclosure, the cells directly have a material to interact with, particularly by attaching to the microcarrier, facilitating cell viability, proliferation, and growth, as well as supporting tissue formation. No additional material engineering, e.g. by coupling cell adhesive moieties to the fiber, is required.

According to some embodiments, the microcarrier and the cells, preferably the microcarrier with the cells attached thereto, and the composition are mixed prior to step (b), such that the fiber comprises a mixture of the microcarrier, the cells and the composition. This allows for spinning in step (b) in a single compound approach, e.g. through one nozzle, spinneret, needle or orifice.

According to some embodiments, step (c) comprises crosslinking or solidifying the complete fiber. This can be advantageous in order to increase the barrier function of the fiber and trap the cells inside. According to some embodiments, the fiber comprises voids or is capable of forming voids allowing the cells to infiltrate, grow and/or differentiate into the voids, preferably wherein voids are formed by phase separation. According to some embodiments, the method comprises forming voids during or subsequently to step (c), preferably forming voids by phase separation. In other or further embodiments, the voids may be formed by temperature shifts. In other or further embodiments, the voids may be formed by leaching a particle which may be co-spun with the other components during spinning step (b). Different techniques to create voids are known by the person skilled in the art and shall not be limiting in scope of the present invention.

### Core-shell hollow fiber

According to particularly preferred embodiments, step (b) comprises spinning the microcarrier and the cells, preferably the microcarrier with the cells attached thereto, and the composition to form a core-shell hollow fiber. Core-shell hollow fibers, particularly wherein the cells and microcarrier are present in the fiber core and the compound capable of crosslinking or solidifying in the fiber shell, are highly advantageous as these allow ample space for cell growth, migration, and spreading in a 3D environment. Surprisingly, the inventors have observed that cells can grow further in empty space of the core of the core-shell hollow fibers where no microcarriers are present. This enables populating the empty space or voids of the core area with cells (see Examples and **Figs. 3-8**). The inventors also observed the ability of the cells growing parallel to the long axis direction of the fibers.

According to preferred embodiments, step (b) comprises spinning using a core-shell nozzle. Such core-shell nozzles are known in the art for various purposes, e.g. textile spinning or electrospinning. Such nozzle allows for delivering one material stream to the fiber shell and another material stream to the fiber core, e.g. delivering the microcarrier and cells to the fiber core and delivering the composition comprising the compound capable of crosslinking or solidifying to the fiber shell. In preferred embodiments when using a core-shell nozzle, the microcarrier and the cells are extruded through the core nozzle, and the composition is extruded through the shell nozzle. According to preferred embodiments when using the core-shell nozzle, it has a core diameter selected from the range of 25 µm to 10,000 µm, 25 µm to 5,000 µm, 50 µm to 4,000 µm, 75 µm to 3,000 µm, 80 µm to 2,000 µm, or 90 µm to 1,500 µm, preferably 100 µm to 1,000 µm, 120 µm to 950 µm, 140 µm to 900 µm, 160 µm to 850 µm, 180 µm to 800 µm, or 190 µm to 750 µm, more preferably 200 µm to 700 µm, and/or a shell diameter selected from the range of 50 µm to 10,000 µm, 100 µm to 9,000 µm, 200 µm to 8,000 µm, 300 µm to 7,000 µm, or 400 µm to 6,000 µm, preferably 500 µm to 5,000 µm, 550 µm to 4,500 µm, 600 µm to 4,000 µm, 600 µm to 3,500 µm, 650 µm to 3,000 µm, 650 µm to 2,500 µm, more preferably 700 µm to 2,000 µm. Such diameters have proven useful and advantageous for producing the core-shell hollow fiber (see Examples, e.g. **Fig. 2**).

According to preferred embodiments, step (c) comprises crosslinking or solidifying the fiber shell and preferably not the fiber core. According to preferred embodiments, step (c) comprises crosslinking or solidifying the fiber shell and not the fiber core. This is advantageous in order to keep the cells in the fiber (at least initially) but at the same time provide the cells in the core a non-crosslinked or non-solidified (micro-)environment such that they have enough space to grow, proliferate and/or differentiate into, enhancing tissue formation. In addition, the fibers are particularly suitable for being applied for artificial meat.

According to preferred embodiments, the fiber is a core-shell hollow fiber, wherein the fiber core comprises the core phase and the fiber shell comprises the shell phase. The core phase may comprise the microcarrier and cells, preferably the cells attached at least partially to the microcarrier. The shell phase may comprise the composition comprising the compound capable of crosslinking or solidifying, wherein such compound may have already crosslinked or solidified as disclosed herein. According to preferred embodiments, the shell of the core-shell hollow fiber crosslinks or solidifies in step (c), preferably by forming a crosslinked polymer matrix.

According to preferred embodiments, the microcarrier, the cells, and the composition are provided separately, such that the generated fiber comprises a core phase comprising the microcarrier and the cells, preferably the microcarrier with the cells attached thereto, and a shell phase comprising the composition. Such configuration has been found useful as is demonstrated in the Examples.

According to preferred embodiments, the fiber shell has a thickness selected from the range of 1 µm to 5,000 µm, 1 µm to 4,000 µm, 1 µm to 3,000 µm, 1 µm to 2,000 µm, 2 µm to 1,750 µm, 3 µm to 1,500 µm, or 4 µm to 1,250 µm, preferably 5 µm to 1,000 µm, 5.5 µm to 900 µm, 6 µm to 800 µm, 6.5 µm to 700 µm, 7 µm to 600 µm, 7.5 µm to 500 µm, 8 µm to 450 µm, 8.5 µm to 400 µm, 9 µm to 350 µm, 9.5 µm to 325 µm, more preferably 10 µm to 300 µm or 20 µm to 100 µm. According to preferred embodiments, the fiber core has a diameter selected from the range of 25 µm to 10,000 µm, 25 µm to 9,000 µm, 25 µm to 8,000 µm, 25 µm to 7,000 µm, 25 µm to 6,000 µm, 25 µm to 5,000 µm, 50 µm to 4,000 µm, 75 µm to 3,000 µm, or 80 µm to 2,000 µm, preferably 100 µm to 1,000 µm, 120 µm to 950 µm, 140 µm to 900 µm, 160 µm to 850 µm, 180 µm to 800 µm, or 190 µm to 750 µm, more preferably 200 µm to 700 µm or 200 µm to 400 µm. Such diameters have proven advantageous for the core-shell hollow fiber (see Examples, **Fig. 3**). These thicknesses and diameters also can mimic natural muscle fibers ranging in diameter from several microns to thousands of microns. Control of shell thickness and core diameter can be achieved by the selected size/diameter of the core-shell nozzle, polymer concertation, as well as the flow rate fed into the core or shell part of nozzle.

According to preferred embodiments, the core of the core-shell hollow fiber comprises voids, preferably wherein the voids are at least partially or essentially fully filled with a liquid. This is particularly advantageous, as this allows the cells to infiltrate and grow into these voids, such that the cells can proliferate facilitating tissue formation. Compared to an inner core that is crosslinked or solidified, it is not required to include particular degradation mechanisms or other techniques for forming the voids such that the cells have space to grow. Hence, this significantly simplifies the fiber production and material engineering. Moreover, in some instances, where the cell adhesive layer in the core is completely degraded or removed, the construct results into a hollow fiber with lumen without any materials for the cells to interact with. This is however important, particularly for cells requiring attachment points, such as muscle cells which are anchorage-dependent type of cells and need a surface to adhere to and expand onto. The microcarrier of the present invention advantageously provides such material to interact with, particularly by providing a surface with which the cells can interact with and/or providing biochemical cues to the cells to improve their growth. The use of microcarrier enables cells to grow in between the voids formed by microcarrier which eliminates the need for sophisticated material design capable of degradation or pore formation. This facilitates cell growth and packed tissue formation within the hollow fibers. Furthermore, the formed tissue can grow anisotropically in direction of the long axis of the hollow fibers which mimics the linear structure of natural muscle tissue. This improves the fiber such that it is particularly suitable for cultured meat applications where a mouthfeel and texture of native meat/muscle are desired.

In some embodiments, the liquid of the voids is suitable for storing and/or culturing the cells at least for a short time, such as at least for 15 min, 30 min, 45 min or 60 min, preferably more than 60 min. This is particularly advantageous, as the cells remain viable in the dispersion or solution during the production of the fiber and afterwards. The liquid may comprise a cell culture medium, preferably it essentially consists of a cell culture medium, more preferably the liquid is a cell culture medium. A cell culture medium is particularly suitable for the production of the fiber to keep the cells viable. The liquid may be thermo-responsive, physically, or chemically crosslinked, or ionically crosslinked. In such embodiments but also in other embodiments, it may be particularly preferred that the liquid is capable of undergoing phase separation, is degradable or non-degradable; and/or is dissolvable and/or degradable, preferably using a chelating agent and/or an enzyme, more preferably a combination of a chelating agent and an enzyme. By allowing some kind of removal of the liquid space can be created for the cells to grow and proliferate and/or provide a fiber from which the liquid can be removed, e.g. in order to produce fiber similar to natural tissue, e.g. such as a muscle fiber, comprising the cells and synthesized ECM.

According to preferred embodiments, the voids provide sufficient space for the cells to proliferate, grow, and/or differentiate into the voids. According to preferred embodiments, at least 1% (v/v) of the core volume are voids, preferably at least 1% (v/v), at least 2% (v/v), at least 5% (v/v), at least 10% (v/v), at least 15% (v/v), at least 20% (v/v), at least 25% (v/v), or at least 30% (v/v) of the core volume are voids, more preferably at least 40% (v/v) or at least 50% (v/v) of the core volume are voids. Such high volumes are advantageous in that these provide sufficient space for the cells to grow into facilitating tissue formation and rendering the fiber very suitable for generating artificial meat.

According to preferred embodiments, the microcarrier is provided at a concentration of least 0.001% (v/v) microcarrier related to total core volume, at least 0.01% (v/v), or at least 0.1% (v/v), preferably at least 1% (v/v) microcarrier related to total core volume, such as at least 2% (v/v) at least 3% (v/v), at least 4% (v/v), at least 5% (v/v), at least 6% (v/v), at least 7% (v/v), at least 8% (v/v), at least 9% (v/v) or 10% (v/v). Such concentrations are useful for providing sufficient microcarrier to interact with the cells. Depending on the microcarrier concentration when being provided in step (a), the microcarrier packing density can be varied and therefore achieve different void volumes within the fiber (between the microcarriers), particularly within the core of the core-shell hollow fiber. The packing density can also be tuned by the core feed rate during spinning step (b), wherein higher feed rate typically leads to more packing and smaller voids. Depending on the distribution of microcarriers within the fiber, preferably within the core of the core-shell hollow fiber, some areas of the fiber, preferably the core, can be without microcarriers, whereas the microcarriers are present in other areas of the fiber.

According to preferred embodiments, the shell of the core-shell hollow fiber is dissolvable or degradable, preferably the shell is dissolved or degraded by the cells throughout cultivation and/or by addition of a degradation agent, such as an enzymatic or chelating degradation agent. As a result, the shell is at least partially, preferably essentially completely, removed when being applied, e.g. for artificial meat, or as medical material, such as engineered tissue for implantation. This can be advantageous in order to produce a cell-containing fiber without material contributed by the shell, preferably a fiber similar to natural tissue, e.g. such as a muscle fiber, containing only the cells and a produced surrounding extracellular matrix (ECM). In such embodiment, the microcarrier may also be degradable or dissolvable, such as enzymatically degradable and/or degradable or dissolvable by a chelating agent. By being degradable or dissolvable, the microcarrier can be removed after fiber production, e.g. by the cells and/or external triggers. As a result, the microcarrier is at least partially, preferably essentially completely, removed to produce a fiber similar to natural tissue, e.g. such as a muscle fiber, without material contributed by the microcarrier (in addition to the shell) for generating said fiber.

### Further embodiments

According to preferred embodiments, prior to step (a) the microcarrier is contacted with the cells and preferably cultured for a sufficient time, such that a microcarrier with cell attached thereto is generated. This is advantageous in order to scale up the cells and use the microcarrier with cells attached thereto as a delivery vehicle for providing step (a). Moreover, such processes are known in the art, such that the cell scale up can utilize existing industry approaches in order to achieve large cell numbers at low cost.

According to preferred embodiments, the method further comprises a step of stretching the fiber after step (b) or during step (c) or after step (c), preferably, during step (c) or after step (c). According to some embodiments, stretching the fiber comprises stretching the fiber through rotational or linear or angular movement of a collector, such as a rotating drum. Fiber stretching advantageously can result into smaller diameter fibers allowing adjustment of the fiber diameter. According to preferred embodiments, the stretching the fiber reduces the fiber diameter, fiber shell thickness, and/or fiber core diameter. Furthermore, fiber stretching advantageously allows for more packed microcarriers in the fiber, e.g. in the fiber core. This can partially align the microcarriers within the fiber, e.g. the fiber core, due to the stretching.

Preferably, the method further comprises a step of culturing the cells of the fiber, preferably after step (c). Culturing of the fibers is particularly advantageous for cells that are alive or mostly viable, such that the culture of the cells allows for maintaining the cells, but also proliferating, facilitating tissue formation. Also, culturing may include a step of differentiating the cells in order to obtain a desired cell type, e.g. pluripotent stem cells or precursor cells into muscle cells or fat cells. Cell growth characteristics can be controlled by the fiber and its filling properties, particularly considering the core of a core-shell hollow fiber. For example, utilizing higher number of microcarriers in the fiber, preferably in the core of the core-shell hollow fiber, advantageously can increase the packing density of the microcarrier and provide more texture to the overall construct. On the other side, higher microcarrier packing density can limit the space for cells to grow in between the voids between the microcarriers, particularly in the core of the core-shell hollow fiber.

According to preferred embodiments, culturing the cells of the fiber is performed for at least 1 h, preferably at least 24 or 48 h, more preferably at least 72 h. In some embodiments, the cells may be cultured longer, such as for at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, or at least 21 days, or longer. The culturing duration may depend on the cell type and/or material of the microcarrier and the compound capable of crosslinking but also the culturing system. Longer culturing duration may in some cases enhance tissue formation producing a fiber similar to natural tissue, e.g. such as a muscle fiber. Shorter culturing duration may be more efficient requiring less resources. Culturing may preferably be performed at least at 4°C or 15°C, preferably at least 25 or 30°C, more preferably at least 35°C, such as 36°C, 37°C, 38°C, 39°C, 40°C, or 41°C. Further suitable culturing conditions for the cell disclosed herein are ready known by the person skilled in the art. In preferred embodiments, culturing is performed by providing a cell culture medium in which the fiber is immersed or in contact with medium. Various culturing techniques and processes are applicable in combination with culturing according to the present invention, including culturing in static or batch, fed-batch and perfusion culture. In many tissue engineering culture approaches, cells are cultured in static or batch culture or perfusion culture. In preferred embodiments, during the culturing step the cells proliferate, grow and/or differentiate, preferably wherein any voids of the fiber provide space for the cells to proliferate, grow, and/or differentiate. The space is advantageously created as disclosed herein between the microcarrier, especially in embodiments, wherein the fiber is a core-shell hollow fiber and the cells and microcarrier are present in the core. The space may also or in addition be created by the composition comprising the compound capable of crosslinking or solidifying, e.g. phase separation, thermo-responsiveness or by including a particle that is sequentially leached. In preferred embodiments, during the culturing step the cells produce an extracellular matrix. This is particularly advantageous in order to form more mature tissue, which particularly closely can resemble or mimic natural tissues, such that this may be particularly advantageous in order to produce artificial meat. In preferred embodiments, during the culturing step the cells degrade the microcarrier and/or the crosslinked or solidified at least part of the fiber. This can be advantageous in order to produce a cell-containing fiber without material contributed by the microcarrier and/or crosslinked at least part of the fiber, preferably a fiber similar to natural tissue, e.g. such as a muscle fiber, containing only the cells and a produced surrounding extracellular matrix (ECM).

According to preferred embodiments, the method comprises a step of dissolving or degrading the microcarrier, preferably after step (c), more preferably after or during a step of culturing the cells of the fiber. According to preferred embodiments, the method comprises a step of dissolving or degrading the crosslinked or solidified at least part of the fiber, preferably after step (c), more preferably after or during a step of culturing the cells of the fiber. According to preferred embodiments, the method comprises after step (c), preferably after or during a step of culturing the cells of the fiber, a step of dissolving or degrading the microcarrier and the crosslinked or solidified at least part of the fiber, such as a shell of a core-shell hollow fiber. According to preferred embodiments, the method comprises adding a degrading agent, preferably an enzymatic or chelating degrading agent for dissolving or degrading the microcarrier and/or the crosslinked or solidified at least part of the fiber. According to preferred embodiments, the method comprises a step of culturing the cells of the fiber, wherein throughout culture the cells dissolve or degrade the microcarrier and/or the crosslinked or solidified at least part of the fiber. Dissolving or degrading the microcarrier and/or the crosslinked or solidified at least part of the fiber can be advantageous in order to produce a cell-containing fiber without material contributed by the microcarrier or and/or the crosslinked or solidified at least part of the fiber. Preferably a fiber similar to natural tissue, e.g. such as a muscle fiber, is produced, containing only the cells and a produced surrounding extracellular matrix (ECM).

According to particular embodiments, spinning in step (b) is performed by wet spinning, wherein the composition further comprises a solvent of the compound, preferably water, optionally wherein the compound is alginate, and in step (c) crosslinking or solidifying is performed by contacting the fiber with a nonsolvent or crosslinking composition, thereby generating the fiber. Such advantageous embodiment leads to desirable properties, particularly for producing artificial meat as demonstrated in the Examples below (see e.g. **Figs. 3-8**).

According to particular embodiments of the method according to the first aspect, the fiber is edible and is a core-shell hollow fiber, wherein the method comprises the following combination of features:
- the microcarrier comprises a cell-adhesive surface, such as a charged surface;
- the cells are animal cells, which are at least partially alive, preferably the cells are one or more cell types found in meat;
- the microcarrier and the cells in step (a) may be provided as one solution or dispersion, wherein at least a part of the cells is attached to the microcarrier;
- spinning step (b) is performed by solution spinning, preferably wet spinning;
- step (b) comprises spinning to form a core-shell hollow fiber, preferably using a core-shell nozzle, wherein the microcarrier and the cells are extruded through the core nozzle, and the composition is extruded through the shell nozzle;
- step (c) comprises crosslinking or solidifying the fiber shell and preferably not the fiber core; and
- the method further comprises a step of culturing the cells of the fiber, preferably after step (c).

The cells according to such embodiments are preferably one or more selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells. In such particular embodiments, step (a) may further comprise providing a microcarrier without cells attached thereto. Preferably, the core of the core-shell hollow fiber comprises voids, more preferably wherein the voids are at least partially or essentially fully filled with a liquid, such as a cell culture medium. The method according to such embodiments, may further comprise a step of stretching the fiber after step (b) or during step (c) or after step (c), preferably, during step (c) or after step (c). Optionally, the microcarrier and/or the crosslinked or solidified at least part of the fiber are degradable or dissolvable, such as enzymatically degradable and/or degradable or dissolvable by a chelating agent. The compound capable of crosslinking or solidifying may be capable of forming a solid matrix, preferably a solid polymeric matrix, optionally, wherein chemical crosslinks and/or physical crosslinks are formed.

According to particular embodiments of the method according to the first aspect, the fiber is edible and is a core-shell hollow fiber, wherein the method comprises the following combination of features:
- the microcarrier comprises a cell-adhesive surface, such as a charged surface;
- the cells are animal cells, which are at least partially alive, preferably the cells are one or more cell types found in meat;
- the microcarrier and the cells in step (a) are provided as separate solutions and/or dispersions;
- spinning step (b) is performed by solution spinning, preferably wet spinning;
- step (b) comprises spinning to form a core-shell hollow fiber, preferably using a core-shell nozzle, wherein the microcarrier and the cells are extruded through the core nozzle, and the composition is extruded through the shell nozzle;
- step (c) comprises crosslinking or solidifying the fiber shell and preferably not the fiber core; and
- the method further comprises a step of culturing the cells of the fiber, preferably after step (c).

The cells according to such embodiments are preferably one or more selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells. In such particular embodiments, step (a) may further comprise providing a microcarrier without cells attached thereto. Preferably, the core of the core-shell hollow fiber comprises voids, more preferably wherein the voids are at least partially or essentially fully filled with a liquid, such as a cell culture medium. The method according to such embodiments, may further comprise a step of stretching the fiber after step (b) or during step (c) or after step (c), preferably, during step (c) or after step (c). Optionally, the microcarrier and/or the crosslinked or solidified at least part of the fiber are degradable or dissolvable, such as enzymatically degradable and/or degradable or dissolvable by a chelating agent. The compound capable of crosslinking or solidifying may be capable of forming a solid matrix, preferably a solid polymeric matrix, optionally, wherein chemical crosslinks and/or physical crosslinks are formed.

According to particular embodiments of the method according to the first aspect, the fiber is edible and is a core-shell hollow fiber, wherein the method comprises the following combination of features:
- the cells are animal cells, which are or become apoptotic or dead, preferably the cells are one or more cell types found in meat;
- the microcarrier and the cells in step (a) are provided as one or separate solution(s) and/or dispersion(s);
- spinning step (b) is performed by melt spinning, extrusion spinning or direct spinning, or solution spinning, such as dry spinning, wet spinning, gel spinning or electrospinning, or a combination thereof, preferably solution spinning, more preferably wet spinning;
- step (b) comprises spinning to form a core-shell hollow fiber, preferably using a core-shell nozzle, wherein the microcarrier and the cells are extruded through the core nozzle, and the composition is extruded through the shell nozzle; and
- step (c) comprises crosslinking or solidifying the fiber shell and preferably not the fiber core.

Such core-shell hollow fibers are directly suitable for consumption without requiring culturing. The cells according to such embodiments are preferably one or more selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells. In such particular embodiments, step (a) may further comprise providing a microcarrier without cells attached thereto. The method according to such embodiments, may further comprise a step of stretching the fiber after step (b) or during step (c) or after step (c), preferably, during step (c) or after step (c). The compound capable of crosslinking or solidifying may be capable of forming a solid matrix, preferably a solid polymeric matrix, optionally, wherein chemical crosslinks and/or physical crosslinks are formed.

According to particular embodiments of the method according to the first aspect, the fiber is suitable for medical purposes and is a core-shell hollow fiber, wherein the method comprises the following combination of features:
- the microcarrier comprises a cell-adhesive surface, such as a charged surface;
- the cells are animal cells, which are at least partially alive, preferably one or more selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells;
- the microcarrier and the cells in step (a) may be provided as one solution or dispersion, wherein at least a part of the cells is attached to the microcarrier;
- spinning step (b) is performed by solution spinning, preferably wet spinning;
- step (b) comprises spinning to form a core-shell hollow fiber, preferably using a core-shell nozzle, wherein the microcarrier and the cells are extruded through the core nozzle, and the composition is extruded through the shell nozzle;
- step (c) comprises crosslinking or solidifying the fiber shell and preferably not the fiber core; and
- the method further comprises a step of culturing the cells of the fiber, preferably after step (c).

In such particular embodiments, step (a) may further comprise providing a microcarrier without cells attached thereto. Preferably, the core of the core-shell hollow fiber comprises voids, more preferably wherein the voids are at least partially or essentially fully filled with a liquid, such as a cell culture medium. The method according to such embodiments, may further comprise a step of stretching the fiber after step (b) or during step (c) or after step (c), preferably, during step (c) or after step (c). Preferably, the microcarrier and/or the crosslinked or solidified at least part of the fiber are degradable or dissolvable, such as enzymatically degradable and/or degradable or dissolvable by a chelating agent. The compound capable of crosslinking or solidifying may be capable of forming a solid matrix, preferably a solid polymeric matrix, optionally, wherein chemical crosslinks and/or physical crosslinks are formed.

According to particular embodiments of the method according to the first aspect, the fiber is suitable for medical purposes and is a core-shell hollow fiber, wherein the method comprises the following combination of features:
- the microcarrier comprises a cell-adhesive surface, such as a charged surface;
- the cells are animal cells, which are at least partially alive, preferably one or more selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells;
- the microcarrier and the cells in step (a) are provided as separate solutions and/or dispersions;
- spinning step (b) is performed by solution spinning, preferably wet spinning;
- step (b) comprises spinning to form a core-shell hollow fiber, preferably using a core-shell nozzle, wherein the microcarrier and the cells are extruded through the core nozzle, and the composition is extruded through the shell nozzle;
- step (c) comprises crosslinking or solidifying the fiber shell and preferably not the fiber core; and
- the method further comprises a step of culturing the cells of the fiber, preferably after step (c).

In such particular embodiments, step (a) may further comprise providing a microcarrier without cells attached thereto. Preferably, the core of the core-shell hollow fiber comprises voids, more preferably wherein the voids are at least partially or essentially fully filled with a liquid, such as a cell culture medium. The method according to such embodiments, may further comprise a step of stretching the fiber after step (b) or during step (c) or after step (c), preferably, during step (c) or after step (c). Preferably, the microcarrier and/or the crosslinked or solidified at least part of the fiber are degradable or dissolvable, such as enzymatically degradable and/or degradable or dissolvable by a chelating agent. The compound capable of crosslinking or solidifying may be capable of forming a solid matrix, preferably a solid polymeric matrix, optionally, wherein chemical crosslinks and/or physical crosslinks are formed.

According to particular embodiments of the method according to the first aspect, the fiber is edible, wherein the method comprises the following combination of features:
- the microcarrier comprises a cell-adhesive surface, such as a charged surface;
- the cells are animal cells, which are at least partially alive, preferably the cells are one or more cell types found in meat;
- the microcarrier and the cells in step (a) may be provided as one solution or dispersion, wherein at least a part of the cells is attached to the microcarrier;
- spinning step (b) is performed by solution spinning, preferably wet spinning;
- step (c) comprises crosslinking or solidifying the complete fiber, wherein the fiber comprises voids or is capable of forming voids allowing the cells to infiltrate, grow and/or differentiate into the voids;
- the compound capable of crosslinking or solidifying is capable of forming a solid matrix, preferably a solid polymeric matrix, optionally, wherein chemical crosslinks and/or physical crosslinks are formed; and
- the method further comprises a step of culturing the cells of the fiber, preferably after step (c).

The cells according to such embodiments are preferably one or more selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells. In such particular embodiments, step (a) may further comprise providing a microcarrier without cells attached thereto. The method according to such embodiments, may further comprise a step of stretching the fiber after step (b) or during step (c) or after step (c), preferably, during step (c) or after step (c). Optionally, the microcarrier and/or the crosslinked or solidified at least part of the fiber are degradable or dissolvable, such as enzymatically degradable and/or degradable or dissolvable by a chelating agent.

According to particular embodiments of the method according to the first aspect, the fiber is edible, wherein the method comprises the following combination of features:
- the microcarrier comprises a cell-adhesive surface, such as a charged surface;
- the cells are animal cells, which are at least partially alive, preferably the cells are one or more cell types found in meat;
- the microcarrier and the cells in step (a) are provided as separate solutions and/or dispersions;
- spinning step (b) is performed by solution spinning, preferably wet spinning;
- step (c) comprises crosslinking or solidifying the complete fiber, wherein the fiber comprises voids or is capable of forming voids allowing the cells to infiltrate, grow and/or differentiate into the voids;
- the compound capable of crosslinking or solidifying is capable of forming a solid matrix, preferably a solid polymeric matrix, optionally, wherein chemical crosslinks and/or physical crosslinks are formed; and
- the method further comprises a step of culturing the cells of the fiber, preferably after step (c).

The cells according to such embodiments are preferably one or more selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells. In such particular embodiments, step (a) may further comprise providing a microcarrier without cells attached thereto. The method according to such embodiments, may further comprise a step of stretching the fiber after step (b) or during step (c) or after step (c), preferably, during step (c) or after step (c). Optionally, the microcarrier and/or the crosslinked or solidified at least part of the fiber are degradable or dissolvable, such as enzymatically degradable and/or degradable or dissolvable by a chelating agent.

According to particular embodiments of the method according to the first aspect, the fiber is edible, wherein the method comprises the following combination of features:
- the cells are animal cells, which are or become apoptotic or dead, preferably the cells are one or more cell types found in meat;
- the microcarrier and the cells in step (a) are provided as one or separate solution(s) and/or dispersion(s);
- spinning step (b) is performed by melt spinning, extrusion spinning or direct spinning, or solution spinning, such as dry spinning, wet spinning, gel spinning or electrospinning, or a combination thereof, preferably solution spinning, more preferably wet spinning;
- step (c) comprises crosslinking or solidifying the complete fiber; and
- the compound capable of crosslinking or solidifying is capable of forming a solid matrix, preferably a solid polymeric matrix, optionally, wherein chemical crosslinks and/or physical crosslinks are formed.

Such fibers are directly suitable for consumption without requiring culturing. The cells according to such embodiments are preferably one or more selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells. In such particular embodiments, step (a) may further comprise providing a microcarrier without cells attached thereto. The method according to such embodiments, may further comprise a step of stretching the fiber after step (b) or during step (c) or after step (c), preferably, during step (c) or after step (c).

### Method for producing a cell-containing core-shell hollow fiber according to a further aspect

According to a further aspect of the invention, a method for producing a cell-containing core-shell hollow fiber is provided, comprising:
(a) providing
   - a microcarrier,
   - cells capable of interacting with the microcarrier, and
   - a composition comprising a compound capable of crosslinking or solidifying;
(b) extruding the microcarrier, the cells and the composition to generate a fiber; and
(c) crosslinking or solidifying at least a part of the fiber.

Such extrusion process may be performed in a spinning process as disclosed herein. Such extrusion process may also be performed in a bioprinting approach, wherein the material is extruded to print the core-shell hollow fiber, wherein such fiber may be one filament of a larger construct suitable for producing a larger 3D structure, preferably for being applied as artificial meat. Preferably in such aspect, step (b) comprises extruding the microcarrier and the cells, preferably the microcarrier with the cells attached thereto, and the composition to form a core-shell hollow fiber. Preferably in such aspect step (b) comprises extruding using a core-shell nozzle, preferably, wherein the microcarrier and the cells are extruded through the core nozzle, and the composition is extruded through the shell nozzle. In preferred embodiments of the aspect, step (c) comprises crosslinking or solidifying the fiber shell and preferably not the fiber core. In embodiments of the aspect, the microcarrier, the cells, and the composition are provided separately, such that the generated fiber comprises a core phase comprising the microcarrier and the cells, preferably the microcarrier with the cells attached thereto, and a shell phase comprising the composition. In preferred embodiments of the aspect, the fiber is a core-shell hollow fiber, wherein the fiber core comprises the core phase and the fiber shell comprises the shell phase.

The method according to the further aspect advantageously allows for producing a cell-containing fiber with improved properties in particular being suitable for creating artificial meat, such as particularly structure meat/muscle or whole meat/muscle (cut). Hence, the above disclosed advantages of the method according to the first aspect can also be found for the method according to the further aspect. Specifically, by providing the microcarrier and cells capable of interacting with the microcarrier, the cells are provided an adequate surrounding to keep them viable and enable proliferation and/or differentiation, e.g. by allowing cells to attach thereto and/or by providing growth factors directly to the cells. In subsequent culture, it was observed that cells can grow further in the empty space of the fiber, particularly the core-shell hollow fiber. This enables populating the whole empty space of the fiber with cells. Moreover, the cells grew parallel to the long axis direction of the fibers, indicating that the cells produce an own aligned ECM similar to native muscle fibers, highlighting the suitability for artificial meat application. Moreover, the extrusion process allows for space between the microcarriers in the fiber for the cells to grow into, but also enables (at least initially) trapping the cells within the fiber, such that the cells are not washed out or released, providing an optimal environment for the cells to grow, and form tissue-like structures. Importantly, the method allows for precisely delivering or seeding the cells in the fiber, achieving desired seeding of the fiber.

The individual steps and preferred embodiments of the method according to the further aspect correspond to the individual steps and embodiments of the method according to the first aspect, except for step (b) as highlighted above. It is referred to the above disclosure which shall equally be applicable for the method according to the further aspect. This particularly but not exclusively includes the cell-containing fiber, the cells, the microcarrier, the composition comprising the compound capable of crosslinking, the core-shell hollow fiber, providing step (a) and crosslinking or solidifying step (c), as well as optional subsequent steps, such as stretching and/or culturing. Further features will now be described in detail.

The fiber according to the further aspect can also have different cross-sectional shapes, including circular, ellipsoidal, triangular, scare, or rectangular. Preferably the cross-sectional shape is essentially or fully isometric. Preferably the fiber cross-sectional shape is ellipsoidal, more preferably circular or essentially circular. The terminology "fiber" is well-known in the field and may interchangeable be used with the term "filament" as is also a well-recognized term in the field of bioprinting. Hence, a fiber in scope of the further aspect may be found equal to a filament as is known in bioprinting.

According to particular embodiments of the method according to the further aspect, the fiber is edible and is a core-shell hollow fiber, wherein the method comprises the following combination of features:
- the microcarrier comprise a cell-adhesive surface, such as a charged surface;
- the cells are animal cells, which are at least partially alive, preferably the cells are one or more cell types found in meat;
- the microcarrier and the cells in step (a) may be provided as one or separate solution(s) or dispersion(s), wherein at least a part of the cells is attached to the microcarrier;
- step (b) comprises extruding to form a core-shell hollow fiber, preferably using a core-shell nozzle, wherein the microcarrier and the cells are extruded through the core nozzle, and the composition is extruded through the shell nozzle;
- step (c) comprises crosslinking or solidifying the fiber shell and preferably not the fiber core; and
- the method further comprises a step of culturing the cells of the fiber, preferably after step (c).

The cells according to such embodiments are preferably one or more selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells. In such particular embodiments, step (a) may further comprise providing a microcarrier without cells attached thereto. Preferably, the core of the core-shell hollow fiber comprises voids, more preferably wherein the voids are at least partially or essentially fully filled with a liquid, such as a cell culture medium. The method according to such embodiments, may further comprise a step of stretching the fiber after step (b) or during step (c) or after step (c), preferably, during step (c) or after step (c). Optionally, the microcarrier and/or the crosslinked or solidified at least part of the fiber are degradable or dissolvable, such as enzymatically degradable and/or degradable or dissolvable by a chelating agent. The compound capable of crosslinking or solidifying may be capable of forming a solid matrix, preferably a solid polymeric matrix, optionally, wherein chemical crosslinks and/or physical crosslinks are formed.

Further encompassed is a cell-containing core-shell hollow fiber, preferably being edible, produced by the method according to the further aspect, preferably wherein the microcarrier and/or the crosslinked or solidified at least part of the fiber are dissolved or degraded. Further encompassed is the edible, cell-containing core-shell hollow fiber, preferably being produced by the method according to the further aspect, for use as artificial meat. Further encompassed is a method for producing artificial meat comprising producing one or more edible, cell-containing core-shell hollow fiber(s) produced by a method according to the further aspect; and collecting and/or assembling the one or more fiber(s) for forming a three-dimensional construct.

### Cell-containing fiber according to the second aspect of the invention

According to a second aspect, a cell-containing fiber, preferably being edible, is provided comprising a microcarrier with cells interacting therewith, wherein at least a part of the fiber is crosslinked or solidified.

The cell-containing fiber according to the second aspect advantageously provides a fiber that is highly suitable for producing artificial meat, particularly for mimicking structured or textured meat. The above disclosed advantages of the method according to the first aspect can also be found for the cell-containing fiber according to the second aspect. Specifically, by containing the microcarrier and cells capable of interacting with the microcarrier, the cells are provided an adequate surrounding to keep them viable and enable proliferation and/or differentiation, e.g. by allowing cells to attach thereto and/or by providing growth factors directly to the cells. On the other side, by being (at least initially) confined within the fiber, the cells are not washed out or released out of the fiber, providing an optimal environment for the cells to grow, and form tissue-like structures. By subjecting the fiber to culture, it was observed that cells can grow further inside the fiber, particularly within the empty space of the fiber, especially for the core-shell hollow fiber (see Examples section). This enables populating the whole empty space of the fiber with cells (see **Fig. 3**). Moreover, the cells grew parallel to the long axis direction of the fibers (see **Figs. 4-8**), indicating that the cells produce an own aligned ECM similar to native muscle fibers, highlighting the suitability for artificial meat application. Furthermore, the cells within the fiber can be adequately supported by nutrients and oxygen, indicating excellent mass transport. The microcarrier additionally provides adequate support for the fiber structure, which is particularly advantageous for partially solidified or crosslinked fibers, such as core-shell hollow fibers, wherein the microcarrier is within the fiber core.

The individual preferred embodiments of the cell-containing fiber according to the second aspect correspond to the individual steps and embodiments of the method according to the first aspect. Therefore, it is referred to the above disclosure which shall equally be applicable for the cell-containing fiber according to the second aspect. This particularly but not exclusively includes the cell-containing fiber, the cells, the microcarrier, the composition comprising the compound capable of crosslinking, the core-shell hollow fiber, providing step (a), spinning step (b), and crosslinking or solidifying step (c), as well as optional subsequent steps, such as stretching and/or culturing. Further features will now be described in detail.

According to preferred embodiments, the fiber is produced by the method according to first aspect of the invention. By utilizing the spinning-based approach as defined in the method according to the first aspect, the fiber can be produced efficiently at large scale and in an inexpensive manner, as well as allowing automation.

According to preferred embodiments, the fiber has one or more of the following characteristics:
(i) the cells are one or more cell types found in meat, preferably one or more cell types found in bovine meat, galline meat, chicken meat, bird meat, goat meat, deer meat, rabbit meat, kangaroo meat, porcine meat, fish meat, and/or ovine meat;
(ii) the cells are selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells and pluripotent stem cells, such as induced pluripotent stem cells or embryonic stem cells, preferably the cells comprise at least a myocyte and/or a muscle satellite cell, optionally further comprising adipocytes, mesenchymal stem/stromal cells, fibro/adipogenic progenitor cells, endothelial cells, and/or pluripotent stem cells;
(iii) the microcarrier is partially or fully covered by cells attached thereto, optionally, wherein the fiber further comprises a microcarrier without cells attached thereto, preferably wherein the ratio of provided microcarrier with cells attached thereto to provided microcarrier without cells attached thereto is between 10:1 to 1:1000, preferably 2:1 to 1:100, more preferably 1:1 to 1:50;
(iv) the microcarrier has an ellipsoidal shape, a spherical shape, a cylindrical shape, a rod-like shape, a cuboid shape, a cone shape, an irregular or random shape, an isotropic shape, or an anisotropic shape, preferably, an ellipsoidal or rod-like shape;
(v) the microcarrier is dispersed in a liquid, preferably the liquid being non-crosslinked, partially crosslinked or crosslinked, more preferably the liquid being non-crosslinked or partially crosslinked; and/or
(vi) the fiber has a diameter selected from the range of 25 µm to 10,000 µm or 50 µm to 5,000 µm, preferably 100 µm to 2,000 µm, more preferably 200 µm to 1,000 µm or 500 µm to 1,000 µm.

According to preferred embodiments, the fiber shell but not the fiber core is crosslinked or solidified. This is advantageous as it allows the cells and microcarrier to remain in the fiber core in a (micro-)environment that may not be crosslinked or solidified and thus provide sufficient space for the cells to grow. At the same time, the cells remain within the fiber, i.e. the fiber continues to contain the cells, as the shell provides (at least initially) a barrier for the cells to lead to some kind of confinement (see Examples, e.g. **Figs. 3-8**).

According to preferred embodiments, the complete fiber is crosslinked or solidified, preferably comprising voids allowing the cells to grow and/or infiltrate into the voids. This can be advantageous in order to increase the barrier function of the fiber and trap the cells inside. On the other side, this can limit the space for the cells to grow, which is why in preferred embodiments, the fiber further contains voids for the cells to grow into. This may be achieved by different techniques, e.g. phase separation, temperature shift, material leaching etc. known in the art.

According to preferred embodiments, the fiber is a core-shell hollow fiber, preferably wherein the fiber shell is crosslinked or solidified not the fiber core, optionally the shell comprising a crosslinked polymer matrix. Preferably the core-shell hollow fiber has one or more of the following characteristics:
(i) the core comprises the microcarrier and cells, preferably the microcarrier with the cells attached thereto;
(ii) the microcarrier is provided at a concentration of least 0.001% (v/v) microcarrier related to total core volume, preferably at least 1% (v/v) or 10% (v/v) microcarrier related to total core volume;
(iii) the fiber shell has a thickness selected from the range of 1 µm to 5,000 µm or 1 µm to 2,000 µm, preferably 5 µm to 1,000 µm, more preferably 10 µm to 300 µm or 20 µm to 100 µm; and/or
(iv) the fiber core has a diameter selected from the range of 25 µm to 10,000 µm or 25 µm to 5,000 µm, preferably 100 µm to 1,000 µm, more preferably 200 µm to 700 µm or 200 µm to 400 µm.

According to preferred embodiments, the core of the core-shell hollow fiber comprises voids, preferably having one or more of the following characteristics:
(i) the voids are at least partially filled with a liquid, such as a cell culture medium;
(ii) the voids provide sufficient space for the cells to proliferate, grow, and/or differentiate into the voids; and/or
(iii) at least 30% (v/v) of the core volume are voids, preferably at least 40% (v/v) or at least 50% (v/v).

### Cell-containing polymer fiber according to the third aspect of the invention

According to a third aspect, a cell-containing fiber, preferably being edible, produced by the method according to the first aspect is provided, wherein the microcarrier and/or the crosslinked or solidified at least part of the fiber are dissolved or degraded.

The cell-containing fiber according to the third aspect advantageously provides a fiber that is highly suitable for producing artificial meat, particularly for mimicking structured or textured meat, but also providing a fiber that comprises less or no material contributed by the microcarrier and/or the crosslinked or solidified at least part of the fiber. This allows for producing a fiber similar to natural tissue, e.g. such as a muscle fiber, containing essentially only the cells and the produced extracellular matrix. In addition, the above disclosed advantages of the cell-containing fiber according to the second aspect can also be found forthe cell-containing fiber according to the third aspect. Importantly, by subjecting the fiber to culture, it was observed that cells can grow further inside the fiber, particularly within the empty space of the fiber, especially for the core-shell hollow fiber (see Examples section). This enables populating the whole empty space of the fiber with cells (see e.g. **Fig. 3**). Moreover, the cells grew parallel to the long axis direction of the fibers (see e.g. **Figs. 4-8**), indicating that the cells produce an own aligned ECM similar to native muscle fibers, highlighting the suitability for artificial meat application. Furthermore, the cells within the fiber can be adequately supported by nutrients and oxygen, indicating excellent mass transport. By utilizing the spinning-based approach as defined in the method according to the first aspect, the fiber can be produced efficiently at large scale and in an inexpensive manner, as well as automation is allowed.

The individual preferred embodiments of the cell-containing fiber according to the third aspect correspond to the individual steps and embodiments of the method according to the first aspect and embodiments of the cell-containing fiber according to the second aspect. Therefore, it is referred to the above disclosure which shall equally be applicable for the cell-containing fiber according to the third aspect. This particularly but not exclusively includes the cell-containing fiber, the cells, the microcarrier, the composition comprising the compound capable of crosslinking, the core-shell hollow fiber, providing step (a), spinning step (b), and crosslinking or solidifying step (c), as well as optional subsequent steps, such as stretching and/or culturing. Further features will now be described in detail.

According to preferred embodiments, the cells of the fiber were cultured, preferably wherein the cells have produced an extracellular matrix. Preferably the extracellular matrix replaces the dissolved or degraded microcarrier and/or the crosslinked or solidified at least part of the fiber. As a result, a fiber can be provided that contains essentially only the cells and its produced extracellular matrix (as well as any culture liquid), which allows for providing a product close to the natural tissue. This is particularly suitable for applications wherein no artificial material should present, including food applications, such as artificial meat, but also medical applications, such as a tissue engineered product to be implanted.

### Use of edible, cell-containing fiber for artificial meat according to the fourth aspect of the invention

According to a fourth aspect, an edible, cell-containing fiber, preferably wherein the fiber is a core-shell hollow fiber, is provided for use as artificial meat. As disclosed above, the edible, cell-containing fiber is highly suitable for producing artificial meat, particularly for mimicking structured or textured meat. The individual preferred embodiments of the edible, cell-containing fiber for use according to the fourth aspect correspond to the individual steps and embodiments of subject matter of the preceding aspects. Therefore, it is referred to the above disclosure which shall equally be applicable for the edible, cell-containing fiber for use according to the fourth aspect. This particularly but not exclusively includes the cell-containing fiber, the cells, the microcarrier, the composition comprising the compound capable of crosslinking, the core-shell hollow fiber, providing step (a), spinning step (b), and crosslinking or solidifying step (c), as well as optional subsequent steps, such as stretching and/or culturing. Further features will now be described in detail.

According to preferred embodiments, the fiber is a fiber according to the second or third aspect of the present invention, preferably produced by a method according to the first aspect of the invention.

According to preferred embodiments, the artificial meat resembles meat selected from one or more of bovine meat, galline meat, chicken meat, bird meat, goat meat, deer meat, rabbit meat, kangaroo meat, porcine meat, fish meat and/or ovine meat. The skilled person will recognize that the present invention shall not be limited to any particular type of meat. For instance, the artificial meat may also resemble mat selected from lion meat, tiger meat, dinosaur meat, mammoth meat, or other less conventional types of meat.

According to preferred embodiments, the fiber is collected or assembled for forming a fiber construct. This is advantageous in order to increase the size and provide a larger construct for consumption.

### Use of cell-containing fiber for medical purpose according to further aspect of the invention

According to a further aspect, a cell-containing fiber, preferably wherein the fiber is a core-shell hollow fiber, is provided for use in medicine, particularly for creating organs for transplantation, engineering tissues for drug testing and disease modeling, promoting wound healing, developing personalized medicine, and advancing cancer research. The individual preferred embodiments of the cell-containing fiber for use according to the further aspect correspond to the individual steps and embodiments of subject matter of the preceding aspects. Therefore, it is referred to the above disclosure which shall equally be applicable forthe cell-containing fiber for use according to the further aspect. This particularly but not exclusively includes the cell-containing fiber, the cells, the microcarrier, the composition comprising the compound capable of crosslinking, the core-shell hollow fiber, providing step (a), spinning step (b), and crosslinking or solidifying step (c), as well as optional subsequent steps, such as stretching and/or culturing. According to preferred embodiments, the fiber is a fiber according to the second or third aspect of the present invention, preferably produced by a method according to the first aspect of the invention.

### System for producing a cell-containing core-shell hollow fiber according to the fifth aspect of the invention

According to a fifth aspect, a spinning system for producing a cell-containing core-shell hollow fiber (4) is provided, wherein the fiber comprises a microcarrier and cells, preferably a microcarrier with cells interacting therewith, the system comprising:
(i) a first container (1) for holding a microcarrier;
(ii) a second container (2) for holding a composition comprising a compound capable of crosslinking or solidifying;
(iii) a core-shell nozzle (3) for extruding the microcarrier through the core nozzle and extruding the composition through the shell nozzle; and
(iv) a collector for collecting the cell-containing core-shell hollow fiber;
wherein the first container (1) further is for holding the cells and/or the system comprises a further container for holding the cells.

The spinning system according to the fifth aspect advantageously allows producing a cell-containing core-shell hollow fiber suitable for producing artificial meat, particularly for mimicking structured or textured meat. The above disclosed advantages of the subject matter according to the preceding aspects can also be found forthe spinning system according to the fifth aspect. In addition, by utilizing the spinning system as defined herein, the cell-containing core-shell hollow fiber can be produced efficiently at large scale and in an inexpensive manner, as well as allowing automation.

The individual preferred embodiments of the spinning system according to the fifth aspect correspond to the individual steps and embodiments of the subject matter according to the preceding aspects. Therefore, it is referred to the above disclosure which shall equally be applicable for the spinning system according to the fifth aspect. This particularly but not exclusively includes the cell-containing fiber, the cells, the microcarrier, the composition comprising the compound capable of crosslinking, the core-shell hollow fiber, providing step (a), spinning step (b), and crosslinking or solidifying step (c), as well as optional subsequent steps, such as stretching and/or culturing. Further features will now be described in detail.

According to preferred embodiments, the core-shell nozzle (3) has:
- a core diameter selected from the range of 25 µm to 700 µm, preferably 100 µm to 500 µm; and/or
- a shell diameter selected from the range of 100 µm to 5,000 µm, preferably 500 µm to 2,000 µm.

According to preferred embodiments when using the core-shell nozzle (3), it has a core diameter selected from the range of 25 µm to 10,000 µm, 25 µm to 5,000 µm, 50 µm to 4,000 µm, 75 µm to 3,000 µm, 80 µm to 2,000 µm, or 90 µm to 1,500 µm, preferably 100 µm to 1,000 µm, 120 µm to 950 µm, 140 µm to 900 µm, 160 µm to 850 µm, 180 µm to 800 µm, or 190 µm to 750 µm, more preferably 200 µm to 700 µm, and/or a shell diameter selected from the range of 50 µm to 10,000 µm, 100 µm to 9,000 µm, 200 µm to 8,000 µm, 300 µm to 7,000 µm, or 400 µm to 6,000 µm, preferably 500 µm to 5,000 µm, 550 µm to 4,500 µm, 600 µm to 4,000 µm, 600 µm to 3,500 µm, 650 µm to 3,000 µm, 650 µm to 2,500 µm, more preferably 700 µm to 2,000 µm. Such diameters have proven useful and advantageous for producing the core-shell hollow fiber (see e.g. Examples, **Fig. 2**).

According to preferred embodiments, the system further comprises one or more of the following:
- a further container for holding a non-solvent or crosslinking composition;
- a source of irradiation, preferably irradiation with light, such as NIR, visible or UV light;
- a temperature control unit;
- a bioreactor for culturing the fiber; and/or
- means for automation, such as a robotic arm or moving stage.

According to preferred embodiments, the collector for collecting the fiber is configured to perform a rotational, linear, angular, vibrational, and/or controlled or random movement, preferably a rotational movement, such as a rotating drum (10). Such collectors are particularly suitable in order to collect and/or assemble the fiber and control the production of larger structures for producing artificial meat. Furthermore, such collectors may advantageously allow stretching of the fiber for controlling the fiber diameter and/or the packing density of the microcarrier in the fiber.

The disclosure further comprises the spinning system for use for performing the method for producing a cell-containing fiber according to the first aspect. The disclosure further comprises the spinning system for use for performing the method for producing artificial meat according to the sixth aspect.

### Method for producing artificial meat according to the sixth aspect of the invention

According to a sixth aspect, a method for producing artificial meat is provided, comprising:
(I) producing one or more edible, cell-containing fiber(s), preferably wherein the fiber(s) are core-shell hollow fiber(s) and/or wherein the fiber(s) are fiber(s) according to the second or third aspect of the invention, preferably produced by a method according to the first aspect of the invention; and
(II) collecting and/or assembling the one or more fiber(s) for forming a three-dimensional construct.

The method for producing artificial meat according to the sixth aspect of the present invention advantageously allows for producing artificial meat, preferably meat having a texture or structure, e.g. whole meat/muscle or whole cut meat/muscle. In particular, by producing one or more edible, cell-containing fiber(s), as disclosed herein and subsequent collecting and/or assembling the one or more fiber(s) the formed three-dimensional construct can resemble meat artificially, especially structured meat. In addition, the above disclosed advantages of preceding aspect of the invention can also be found for the method according to the sixth aspect. Importantly, the present invention allows for precisely delivering or seeding the cells in the fiber, allowing for a desired seeding of the fiber, as well as larger constructs for producing artificial meat. Furthermore, by subjecting the three-dimensional construct to culture, it was observed that cells can grow further inside the fiber(s) of the construct, particularly within the empty space of the fiber, especially for the core-shell hollow fiber (see Examples). This enables populating the whole empty space of the fiber with cells (see e.g. **Fig. 3**). Moreover, the cells grew parallel to the long axis direction of the fibers (see e.g. **Figs. 4-8**), indicating that the cells produce an own aligned ECM similar to native muscle fibers, highlighting the suitability for artificial meat application. Furthermore, the cells within the fiber can be adequately supported by nutrients and oxygen, indicating excellent mass transport.

The individual preferred embodiments of the method according to the sixth aspect correspond to the individual steps and embodiments of the method according to the first aspect concerning step (I) but also for the cell-containing fibers according to the second and third aspect. Therefore, it is referred to the above disclosure which shall equally be applicable for the method according to the sixth aspect. This particularly but not exclusively includes the cell-containing fiber, the cells, the microcarrier, the composition comprising the compound capable of crosslinking, the core-shell hollow fiber, providing step (a), spinning step (b), and crosslinking or solidifying step (c), as well as optional subsequent steps, such as stretching and/or culturing. Further features will now be described in detail.

According to preferred embodiments, collecting and/or assembling in step (II) further comprises stretching the one or more fiber(s), wherein preferably stretching comprises stretching the one or more fibers through rotational movement of a collector, such as a rotating drum. Fiber stretching advantageously can result into smaller diameter fibers allowing adjustment of the fiber diameter. According to preferred embodiments, the stretching the fiber reduces the fiber diameter, fiber shell thickness, and/or fiber core diameter. Furthermore, fiber stretching advantageously allows for more packed microcarriers in the fiber, e.g. in the fiber core. This can partially align the microcarriers within the fiber, e.g. the fiber core, due to the stretching.

According to preferred embodiments, collecting and/or assembling in step (II) comprises assembling the one or more fiber(s) longitudinally predominantly in one direction, preferably assembling essentially parallel. Such assembly and/or collection is particularly suitable to produce artificial meat with texture mimicking traditional meat at large scale. Thereby, the method achieves a realistic texture in tissue-engineered meat which is crucial for consumer acceptance, enhancing the overall sensory experience of eating, promoting market adoption, enabling diverse culinary applications, and positively influencing the perceived nutritional value of the product.

The collecting and/or assembling in step (II) may preferably comprise assembling two or more layers of cell-containing fibers, preferably core-shell hollow fibers, particularly on top of each other, for forming a three-dimensional construct. Such constructs are particularly suitable for producing an edible texturized meat like product comprising two or more layers of fibrous elements, wherein each fiber comprises microcarriers and one or more cell types, wherein different fibers may comprise the same or different cell types.

According to preferred embodiments, step (I) comprises producing a plurality of edible, cell-containing fibers. Such plurality may comprise providing fibers comprising the same or different cells. For instance, it may be desirable to provide one cell type or a mixture of cell types in one fiber and provide multiple of such fibers, simplifying the overall method. It may also be desirable to provide fibers or tissues, wherein some fibers comprise different cell types in order to better mimic the natural meat. For instance, fibers comprising myocytes or satellite cells may be combined with fibers comprising adipocytes or progenitors thereof. In addition, fibers comprising endothelial cells may be provided. As a result, providing some fibers comprising different cell types, it is possible to resemble the marbling, which is referred to the intramuscular fat distribution within meat. Producing these at large mass scales, is advantageous in tissue-engineered meat production for meeting consumer demand and widespread market accessibility, cost efficiency, and potential environmental benefits.

According to preferred embodiments, the method further comprises step (III) culturing the cells of the three-dimensional construct. Culturing of the three-dimensional construct is particularly advantageous for cells that are alive or mostly viable, such that the culture of the cells allows for maintaining the cells, but also proliferating, facilitating tissue formation. Also, culturing may include a step of differentiating the cells in order to differentiate the cells into a desired cell type, e.g. pluripotent stem cells or precursor cells into muscle cells or fat cells. Cell growth characteristics can be controlled by the fiber and its filling properties, particularly considering the core of a core-shell hollow fiber. For example, utilizing higher number of microcarriers in the fiber, preferably in the core of the core-shell hollow fiber, advantageously can increase the packing density of the microcarrier and provide more texture to the overall construct. On the other side, higher microcarrier packing density can limit the space for cells to grow in between the voids between the microcarriers, particularly in the core of the core-shell hollow fiber.

According to preferred embodiments, culturing step (III) is performed for at least 1 h, preferably at least 24 or 48 h, more preferably at least 72 h. In some embodiments, the cells may be cultured longer, such as for at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, or at least 21 days, or longer. The culturing duration may depend on the cell type and/or material of the microcarrier and the compound capable of crosslinking but also the culturing system. Longer culturing duration may in some cases enhance tissue formation for producing three-dimensional constructs similar to natural tissue, e.g. such as muscle tissues including muscle fibers. Shorter culturing duration may be more efficient requiring less resources. Culturing may preferably be performed at least at 4°C or 15°C, preferably at least 25 or 30°C, more preferably at least 35°C, such as 36°C, 37°C, 38°C, 39°C, 40°C, or 41 °C. Further suitable culturing conditions for the cell disclosed herein are ready known by the person skilled in the art. In preferred embodiments, culturing is performed by providing a cell culture medium in which the fiber is immersed or in contact with medium. Various culturing techniques and processes are applicable in combination with culturing according to the present invention, including culturing in static or batch, fed-batch, and perfusion culture. In many tissue engineering culture approaches, cells are cultured in static or batch culture or perfusion culture.

In preferred embodiments, during the culturing step (III) the cells proliferate, grow and/or differentiate, preferably wherein any voids of the fiber provide space for the cells to proliferate, grow, and/or differentiate. The space is advantageously created as disclosed herein between the microcarrier, especially in embodiments, wherein the fiber is a core-shell hollow fiber and the cells and microcarrier are present in the core. The space may also or in addition be created by the composition comprising the compound capable of crosslinking or solidifying, e.g. phase separation, thermo-responsiveness or by including a particle that is sequentially leached. In preferred embodiments, during the culturing step the cells produce an extracellular matrix. This is particularly advantageous in order to form more mature tissue, which particularly closely can resemble or mimic natural tissues, such that this may be particularly advantageous in order to produce artificial meat. In preferred embodiments, during the culturing step the cells degrade the microcarrier and/or the crosslinked or solidified at least part of the fiber. This can be advantageous in order to produce a cell-containing fiber without material contributed by the microcarrier and/or crosslinked at least part of the fiber, preferably a fiber similar to natural tissue, e.g. such as a muscle fiber, containing only the cells and a produced surrounding extracellular matrix (ECM).

According to preferred embodiments, the method comprises a step of dissolving or degrading the microcarrier, preferably after step (II), more preferably after or during a step of culturing the cells of the fiber(s). According to preferred embodiments, the method comprises a step of dissolving or degrading the crosslinked or solidified at least part of the fiber(s), preferably after step (II), more preferably after or during a step of culturing the cells of the fiber(s). According to preferred embodiments, the method comprises after step (II), preferably after or during a step of culturing the cells of the fiber(s), a step of dissolving or degrading the microcarrier and the crosslinked or solidified at least part of the fiber(s), such as a shell of core-shell hollow fiber(s). According to preferred embodiments, the method comprises adding a degrading agent, preferably an enzymatic or chelating degrading agent for dissolving or degrading the microcarrier and/or the crosslinked or solidified at least part of the fiber(s). According to preferred embodiments, the method comprises a step of culturing the cells of the fiber(s), wherein throughout culture the cells dissolve or degrade the microcarrier and/or the crosslinked or solidified at least part of the fiber(s). Dissolving or degrading the microcarrier and/or the crosslinked or solidified at least part of the fiber can be advantageous in order to produce a cell-containing fiber without material contributed by the microcarrier or and/or the crosslinked or solidified at least part of the fiber. Preferably, a fiber similar to natural tissue, e.g. such as a muscle fiber, is produced, containing only the cells and a produced surrounding extracellular matrix (ECM).

According to particular embodiments of the method according to the sixth aspect, the fiber(s) of step (I) is/are edible and is/are core-shell hollow fiber(s), having one or more, preferably all of the following characteristics:
- the microcarrier comprise a cell-adhesive surface, such as a charged surface;
- the cells are animal cells, which are at least partially alive, preferably the cells are one or more cell types found in meat, optionally wherein the different cell types are provided in the fiber(s); and/or
- the generated fiber(s) comprise(s) a core phase comprising the microcarrier and the cells, preferably the microcarrier with the cells attached thereto, and a shell phase comprising the composition comprising the compound capable of crosslinking or solidifying.

The cells according to such embodiments are preferably one or more selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells. Preferably, the core of the core-shell hollow fiber comprises voids, more preferably wherein the voids are at least partially or essentially fully filled with a liquid, such as a cell culture medium. The method according to such embodiments, may in collecting and/or assembling step (II) further comprise stretching the one or more fiber(s), wherein preferably stretching comprises stretching the one or more fibers through rotational movement of a collector, such as a rotating drum. The method according to such embodiments, may further comprises step (III) culturing the cells of the three-dimensional construct. Optionally, the microcarrier and/or the crosslinked or solidified at least part of the fiber are degradable or dissolvable, such as enzymatically degradable and/or degradable or dissolvable by a chelating agent.

### Artificial meat according to the seventh aspect of the invention

According to a seventh aspect, artificial meat is provided comprising assembled and/or collected one or more edible, cell-containing fiber(s). Preferably the fiber(s) are core-shell hollow fiber(s). In a particularly preferred embodiment, the fiber(s) are fiber(s) according to the second or third aspect of the invention. The fiber(s) may be advantageously produced by a method according to the first aspect of the invention. According to preferred embodiments, the artificial meat is produced by a method according to the sixth aspect of the invention.

The artificial meat according to the seventh aspect of the invention advantageously embodies the advantages disclosed above for the aspects one to six of the invention. Hence, the above disclosed advantages can also be found in the artificial meat according to the seventh aspect. The individual and preferred embodiments of the artificial meat according to the seventh aspect correspond to the embodiments of the first to sixth aspect. Therefore, it is referred to the above disclosure which shall equally be applicable forthe artificial according to seventh aspect. This particularly but not exclusively includes the cell-containing fiber, the cells, the microcarrier, the composition comprising the compound capable of crosslinking, the core-shell hollow fiber, providing step (a), spinning step (b), and crosslinking or solidifying step (c), as well as optional subsequent steps, such as stretching and/or culturing.

### Items according to the present disclosure

The following items provide further advantageous embodiments of the present disclosure:
1. A method for producing a cell-containing fiber comprising:
   (a) providing
      - a microcarrier,
      - cells capable of interacting with the microcarrier, and
      - a composition comprising a compound capable of crosslinking or solidifying;
   (b) spinning the microcarrier, the cells and the composition to generate a fiber; and
   (c) crosslinking or solidifying at least a part of the fiber.
2. The method according to item 1, wherein the fiber is edible.
3. The method according to item 1 or 2, wherein the fiber is suitable for medical purposes.
4. The method according to one or more of items 1 to 3, wherein the microcarrier and the cells in step (a) are provided as one solution or dispersion, wherein at least a part of the cells is attached to the microcarrier.
5. The method according to one or more of items 1 to 4, wherein the microcarrier and the cells in step (a) are provided as separate solutions and/or dispersions.
6. The method according to one or more of items 1 to 5, wherein the fiber is a polymeric fiber, preferably wherein crosslinking or solidifying at least a part of the fiber in step (c) comprises a crosslinked or solidified polymer matrix of at least a part of the fiber.
7. The method according to one or more of items 1 to 6, wherein the cells are eukaryotic cells, preferably, animal cells.
8. The method according to one or more of items 1 to 7, wherein the cells are one or more cell types found in meat, preferably one or more cell types found in bovine meat, galline meat, chicken meat, bird meat, goat meat, deer meat, rabbit meat, kangaroo meat, porcine meat, fish meat and/or ovine meat.
9. The method according to one or more of items 1 to 8, wherein the cells are selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells.
10. The method according to one or more of items 1 to 9, wherein the cells comprise at least a myocyte and/or a muscle satellite cell, optionally further comprising adipocytes, mesenchymal stem/stromal cells, fibro/adipogenic progenitor cells, endothelial cells, and/or pluripotent stem cells.
11. The method according to one or more of items 1 to 10, wherein the cells are alive, apoptotic and/or dead.
12. The method according to one or more of items 1 to 11, wherein the cells are differentiated cells, non-differentiated cells, pluripotent cells, multipotent cells, reprogrammed cells, or a combination thereof.
13. The method according to one or more of items 1 to 12, wherein the cells are capable of interacting with the microcarrier by being capable of (i) attaching to the microcarrier and/or (ii) sensing chemical or biochemical cues originating from the microcarrier.
14. The method according to one or more of items 1 to 13, wherein the microcarrier comprises a surface that is suitable for the cells to attach thereto, preferably suitable for anchorage-dependent cells, and/or wherein the microcarrier comprises a cell-adhesive surface.
15. The method according to item 14, wherein the surface of the microcarrier has one or more of the following characteristics:
   - it is charged, such as positively or negatively charged;
   - it has moderate hydrophilicity, such as 20 to 40° water contact angle;
   - it is rough, porous or structured; and/or
   - it comprises a cell adhesive moiety, preferably a moiety recognized by one or more cellular integrins, such as a moiety selected from one or more of laminin, fibronectin, collagen, gelatin, vitronectin or fragments or peptides thereof, including RGD or IKVAV or a peptide sequence comprising these.
16. The method according to one or more of items 1 to 15, wherein the microcarrier is partially or fully covered by cells attached thereto.
17. The method according to one or more of items 1 to 16, wherein step (a) further comprises providing a microcarrier without cells attached thereto, preferably wherein the volume or mass ratio of provided microcarrier with cells attached thereto to provided microcarrier without cells attached thereto is between 10:1 to 1:1000, preferably 2:1 to 1:100, more preferably 1:1 to 1:100 or 1:1 to 1:50.
18. The method according to one or more of items 1 to 17, wherein the microcarrier material is edible and/or suitable for medical purposes, particularly selected from one or more of:
   (i) monosaccharides, oligosaccharides or polysaccharides, such as alginate, cellulose, chitosan, hyaluronan, agar, inulin, pectin, methyl cellulose, dextran, tapioca, xanthan gum, guar gum, tara gum, bean gum, starch, gum Arabic, carboxymethyl amylose, carboxymethyl chitosan, chrondoitin sulfate, dermatan sulfate, heparin, heparin sulfate, natural mixes, including corn syrup, honey, maple syrup, or glucose syrup, or a combination thereof, preferably polysaccharides, more preferably, alginate, pectin, methyl cellulose, chitosan, dextran, or a combination thereof;
   (ii) proteins, preferably gelatin, collagen, fibrinogen, fibrin, fibronectin, elastin, laminin, soy protein, zein protein, pea protein, canola protein, carob protein, cardosine A, wheat protein, albumin, casein protein, potato protein, suar protein, gluten, legume protein, corn protein, sorghum protein, animal protein, animal protein isolate, beef protein isolate, whey protein;
   (iii) sugar alcohols;
   (iv) protein hydrolysates; or
   (v) a combination of any of (i) to (iv).
19. The method according to one or more of items 1 to 18, wherein the microcarrier has an ellipsoidal shape, a spherical shape, a cylindrical shape, a rod-like shape, a cuboid shape, a cone shape, an irregular or random shape, an isotropic shape, or an anisotropic shape, preferably, an ellipsoidal or rod-like shape.
20. The method according to one or more of items 1 to 19, wherein the microcarrier is porous, non-porous or hollow.
21. The method according to one or more of items 1 to 20, wherein the microcarrier is degradable or dissolvable, such as enzymatically degradable and/or degradable or dissolvable by a chelating agent.
22. The method according to one or more of items 1 to 21, wherein the microcarrier has a size selected from 10 µm to 1,000 µm, preferably 50 µm to 500 µm, more preferably 100 µm to 300 µm.
23. The method according to one or more of items 1 to 22, wherein the microcarrier is dispersed in a liquid, preferably the liquid being non-crosslinked, partially crosslinked or crosslinked, more preferably the liquid being non-crosslinked or partially crosslinked.
24. The method according to item 23, wherein the liquid has one of more of the following characteristics:
   - it is suitable for storing and/or culturing the cells at least for a short time, such as at least for 15 min, 30 min, 45 min or 60 min, preferably more than 60 min;
   - it comprises a cell culture medium, preferably it essentially consists of a cell culture medium, more preferably the liquid is a cell culture medium;
   - it is thermo-responsive;
   - it is physically or chemically crosslinked;
   - it is ionically crosslinked;
   - it is capable of undergoing phase separation;
   - it is degradable or non-degradable; and/or
   - it is dissolvable and/or degradable, preferably using a chelating agent and/or an enzyme, more preferably a combination of a chelating agent and an enzyme.
25. The method according to one or more of items 1 to 24, wherein spinning step (b) is performed by a process selected from
   (i) melt spinning, extrusion spinning or direct spinning; or
   (ii) solution spinning, such as dry spinning, wet spinning, gel spinning or electrospinning; or
   (iii) a combination of any process of (i) and/or (ii); or
   (iv) preferably solution spinning, more preferably wet spinning.
26. The method according to one or more of items 1 to 25, wherein the fiber has a diameter selected from the range of 25 µm to 10,000 µm or 50 µm to 5,000 µm, preferably 100 µm to 2,000 µm, more preferably 200 µm to 1,000 µm or 500 µm to 1,000 µm.
27. The method according to one or more of items 1 to 26, wherein the compound capable of crosslinking or solidifying is capable of forming a solid matrix, preferably a solid polymeric matrix, optionally, wherein chemical crosslinks and/or physical crosslinks are formed.
28. The method according to one or more of items 1 to 27, wherein the compound is a monomer, prepolymer or polymer, preferably a polymer.
29. The method according to one or more of items 1 to 28, wherein the compound is edible and/or suitable for medical purposes, particularly selected from one or more of
   (i) monosaccharides, oligosaccharides or polysaccharides, such as alginate, cellulose, chitosan, hyaluronan, agar, inulin, pectin, methyl cellulose, dextran, tapioca, xanthan gum, guar gum, tara gum, bean gum, starch, gum Arabic, carboxymethyl amylose, carboxymethyl chitosan, chrondoitin sulfate, dermatan sulfate, heparin, heparin sulfate, natural mixes, including corn syrup, honey, maple syrup, or glucose syrup, or a combination thereof, preferably polysaccharides, more preferably, alginate, pectin, methyl cellulose, chitosan, dextran, or a combination thereof;
   (ii) proteins, preferably gelatin, collagen, fibrinogen, fibrin, fibronectin, elastin, laminin, soy protein, zein protein, pea protein, canola protein, carob protein, cardosine A, wheat protein, albumin, casein protein, potato protein, suar protein, gluten, legume protein, corn protein, sorghum protein, animal protein, animal protein isolate, beef protein isolate, whey protein;
   (iii) sugar alcohols;
   (iv) protein hydrolysates; or
   (v) a combination of any of (i) to (iv).
30. The method according to one or more of items 1 to 29, wherein the composition provided in step (a) further comprises a liquid in which the compound is dissolved or dispersed, preferably the liquid is selected from water, including tap water, pure water, distilled water, deionized water, RO water, RO-EDI water, ultrapure water, cell culture medium, phosphate-buffered saline (PBS), physiological saline or the like, with water being preferred.
31. The method according to one or more of items 1 to 30, wherein the compound capable of crosslinking or solidifying has one of more of the following characteristics:
   - it can be physically or chemically crosslinked;
   - it can be ionically crosslinked;
   - it is thermo-responsive;
   - it can undergo phase separation;
   - it is degradable or non-degradable;
   - it is dissolvable and/or degradable, preferably using a chelating agent and/or an enzyme, more preferably a combination of a chelating agent and an enzyme; and/or
   - it is biocompatible.
32. The method according to one or more of items 1 to 31, wherein the composition provided in step (a) is extrudable through a nozzle for spinning the fiber in step (b).
33. The method according to one or more of items 1 to 32, wherein the composition provided in step (a) is liquid or becomes liquid upon spinning the fiber in step (b).
34. The method according to one or more of items 1 to 33, wherein crosslinking or solidifying at least a part of the fiber in step (c) comprises subjecting the fiber to one or more of the following:
   - a nonsolvent of the compound capable of crosslinking or solidifying;
   - a change in one or more conditions, preferably a change in temperature, humidity, shear forces, or pressure;
   - a crosslinking composition comprising one or more crosslinking reagents for crosslinking the compound capable of crosslinking or solidifying, preferably to form a solid polymer matrix, more preferably the crosslinking composition comprises ions for forming ionic crosslinks;
   - one or more enzyme(s), optionally including further co-factors or compounds required for the enzymatic reaction, or
   - irradiation, preferably irradiation with light, such as NIR, visible or UV light.
35. The method according to one or more of items 1 to 34, wherein a further composition is provided capable of reacting with the compounds capable of crosslinking or solidifying, wherein the composition and the further composition are contacted prior to, during or after spinning step (b), preferably wherein during or after spinning step (b), and at least part of the fiber undergoes crosslinking or solidifying of step (c).
36. The method according to one or more of items 1 to 35, wherein spinning in step (b) is performed by wet spinning, wherein the composition further comprises a solvent of the compound, preferably water, optionally wherein the compound is alginate, and in step (c) crosslinking or solidifying is performed by contacting the fiber with a nonsolvent or crosslinking composition, thereby generating the fiber.
37. The method according to one or more of items 1 to 36, wherein step (c) comprises crosslinking or solidifying
   (i) the fiber shell, preferably not the fiber core; or
   (ii) the complete fiber.
38. The method according to one or more of items 1 to 37, wherein the microcarrier and the cells, preferably the microcarrier with the cells attached thereto, and the composition are mixed prior to step (b), such that the fiber comprises a mixture of the microcarrier, the cells and the composition.
39. The method according to item 38, wherein step (c) comprises crosslinking or solidifying the complete fiber.
40. The method according to item 38 or 39, wherein the fiber comprises voids or is capable of forming voids allowing the cells to infiltrate, grow and/or differentiate into the voids, preferably wherein voids are formed by phase separation.
41. The method according to one or more of items 38 to 40, wherein the method comprises forming voids during or subsequently to step (c), preferably forming voids by phase separation.
42. The method according to one or more of items 1 to 37, wherein step (b) comprises spinning the microcarrier and the cells, preferably the microcarrier with the cells attached thereto, and the composition to form a core-shell hollow fiber.
43. The method according to one or more of items 1 to 37 or 42, wherein step (b) comprises spinning using a core-shell nozzle, preferably, wherein
   - the microcarrier and the cells are extruded through the core nozzle, and
   - the composition is extruded through the shell nozzle.
44. The method according to item 43, wherein the core-shell nozzle has:
   - a core diameter selected from the range of 25 µm to 10,000 µm or 25 µm to 5,000 µm, preferably 100 µm to 1,000 µm, more preferably 200 µm to 700 µm; and/or
   - a shell diameter selected from the range of 50 µm to 10,000 µm, preferably 500 µm to 5,000 µm, more preferably 700 µm to 2,000 µm.
45. The method according to one or more of items 42 to 44, wherein step (c) comprises crosslinking or solidifying the fiber shell and preferably not the fiber core.
46. The method according to one or more of items 42 to 45, wherein the microcarrier, the cells, and the composition are provided separately, such that the generated fiber comprises
   - a core phase comprising the microcarrier and the cells, preferably the microcarrier with the cells attached thereto, and
   - a shell phase comprising the composition.
47. The method according to item 46, wherein the fiber is a core-shell hollow fiber, wherein the fiber core comprises the core phase and the fiber shell comprises the shell phase.
48. The method according to one or more of items 42 to 47, wherein the fiber shell has a thickness selected from the range of 1 µm to 5,000 µm or 1 µm to 2,000 µm, preferably 5 µm to 1,000 µm, more preferably 10 µm to 300 µm or 20 µm to 100 µm.
49. The method according to one or more of items 42 to 48, wherein the fiber core has a diameter selected from the range of 25 µm to 10,000 µm or 25 µm to 5,000 µm, preferably 100 µm to 1,000 µm, more preferably 200 µm to 700 µm or 200 µm to 400 µm.
50. The method according to one or more of items 42 to 49, wherein the core of the core-shell hollow fiber comprises voids, preferably wherein the voids are at least partially or essentially fully filled with a liquid, optionally the liquid having one of more of the following characteristics:
   - it is suitable for storing and/or culturing the cells at least for a short time, such as at least for 15 min, 30 min, 45 min or 60 min, preferably more than 60 min;
   - it comprises a cell culture medium, preferably it essentially consists of a cell culture medium, more preferably the liquid is a cell culture medium;
   - it is thermo-responsive;
   - it is physically or chemically crosslinked;
   - it is ionically crosslinked;
   - it is capable of undergoing phase separation;
   - it is degradable or non-degradable; and/or
   - it is dissolvable and/or degradable, preferably using a chelating agent and/or an enzyme, more preferably a combination of a chelating agent and an enzyme.
51. The method according to item 50, wherein the voids provide sufficient space for the cells to proliferate, grow, and/or differentiate into the voids.
52. The method according to item 50 or 51, wherein at least 1% (v/v) of the core volume are voids, preferably at least 1% (v/v), at least 2% (v/v), at least 5% (v/v), at least 10% (v/v), at least 15% (v/v), at least 20% (v/v), at least 25% (v/v), or at least 30% (v/v) of the core volume are voids, more preferably at least 40% (v/v) or at least 50% (v/v) of the core volume are voids.
53. The method according to one or more of items 42 to 52, wherein the microcarrier is provided at a concentration of least 0.001% (v/v) microcarrier related to total core volume, preferably at least 1% (v/v) or 10% (v/v) microcarrier related to total core volume.
54. The method according to one or more of items 42 to 53, wherein the shell of the core-shell hollow fiber crosslinks or solidifies in step (c), preferably by forming a crosslinked polymer matrix.
55. The method according to one or more of items 42 to 54, wherein the shell of the core-shell hollow fiber is dissolvable or degradable, preferably the shell is dissolved or degraded by the cells throughout cultivation and/or by addition of a degradation agent, such as an enzymatic or chelating degradation agent.
56. The method according to one or more of items 1 to 55, wherein the method further comprises a step of stretching the fiber after step (b) or during step (c) or after step (c), preferably, during step (c) or after step (c), optionally, wherein stretching the fiber comprises stretching the fiber through rotational or linear or angular movement of a collector, such as a rotating drum.
57. The method according to item 56, wherein the stretching the fiber reduces the fiber diameter, fiber shell thickness, and/or fiber core diameter.
58. The method according to one or more of items 1 to 57, wherein the method further comprises a step of culturing the cells of the fiber, preferably after step (c).
59. The method according to item 58, wherein the step of culturing the cells of the fiber has one or more of the following characteristics:
   - culturing is performed for at least 1 h, preferably at least 24 or 48 h, more preferably at least 72 h;
   - culturing is performed at least at 4°C or 15°C, preferably at least 25 or 30°C, more preferably at least 35°C, such as 36°C, 37°C, 38°C, 39°C, 40°C, or 41 °C;
   - culturing is performed by providing a cell culture medium in which the fiber is immersed or in contact with medium;
   - during the culturing step the cells proliferate, grow and/or differentiate, preferably wherein any voids of the fiber provide space for the cells to proliferate, grow, and/or differentiate;
   - during the culturing step the cells produce an extracellular matrix; and/or
   - during the culturing step the cells degrade the microcarrier and/or the crosslinked or solidified at least part of the fiber.
60. The method according to one or more of items 1 to 59, wherein prior to step (a) the microcarrier is contacted with the cells and preferably cultured for a sufficient time, such that the microcarrier with cell attached thereto is generated.
61. The method according to one or more of items 1 to 60, wherein the method has one or more of the following characteristics:
   (i) it comprises a step of dissolving or degrading the microcarrier, preferably after step (c), more preferably after or during a step of culturing the cells of the fiber;
   (ii) it comprises a step of dissolving or degrading the crosslinked or solidified at least part of the fiber, preferably after step (c), more preferably after or during a step of culturing the cells of the fiber;
   (iii) it comprises after step (c), preferably after or during a step of culturing the cells of the fiber, a step of dissolving or degrading the microcarrier and the crosslinked or solidified at least part of the fiber, such as a shell of a core-shell hollow fiber;
   (iv) it comprises adding a degrading agent, preferably an enzymatic or chelating degrading agent for dissolving or degrading the microcarrier and/or the crosslinked or solidified at least part of the fiber; and/or
   (v) it comprises a step of culturing the cells of the fiber, wherein throughout culture the cells dissolve or degrade the microcarrier and/or the crosslinked or solidified at least part of the fiber.
62. A cell-containing fiber, preferably being edible, comprising a microcarrier with cells interacting therewith, wherein at least a part of the fiber is crosslinked or solidified.
63. The fiber according to item 62, wherein the fiber is produced by the method according to one or more of items 1 to 61.
64. The fiber according to item 62 or 63, wherein the fiber has one or more of the following characteristics:
   (i) the cells are one or more cell types found in meat, preferably one or more cell types found in bovine meat, galline meat, chicken meat, bird meat, goat meat, deer meat, rabbit meat, kangaroo meat, porcine meat, fish meat, and/or ovine meat;
   (ii) the cells are one or more cell types found in an animal, preferably one or more cell types isolated or derived from bovine cells, galline cells, chicken cells, bird cells, goat cells, deer cells, rabbit cells, kangaroo cells, porcine cells, fish cells and/or ovine cells;
   (iii) the cells are selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells and pluripotent stem cells, such as induced pluripotent stem cells or embryonic stem cells, preferably the cells comprise at least a myocyte and/or a muscle satellite cell, optionally further comprising adipocytes, mesenchymal stem/stromal cells, fibro/adipogenic progenitor cells, endothelial cells, and/or pluripotent stem cells;
   (iv) the microcarrier is partially or fully covered by cells attached thereto, optionally, wherein the fiber further comprises a microcarrier without cells attached thereto, preferably wherein the ratio of provided microcarrier with cells attached thereto to provided microcarrier without cells attached thereto is between 10:1 to 1:1000, preferably 2:1 to 1:100, more preferably 1:1 to 1:50;
   (v) the microcarrier has an ellipsoidal shape, a spherical shape, a cylindrical shape, a rod-like shape, a cuboid shape, a cone shape, an irregular or random shape, an isotropic shape, or an anisotropic shape, preferably, an ellipsoidal or rod-like shape;
   (vi) the microcarrier is dispersed in a liquid, preferably the liquid being non-crosslinked, partially crosslinked or crosslinked, more preferably the liquid being non-crosslinked or partially crosslinked; and/or
   (vii) the fiber has a diameter selected from the range of 25 µm to 10,000 µm or 50 µm to 5,000 µm, preferably 100 µm to 2,000 µm, more preferably 200 µm to 1,000 µm or 500 µm to 1,000 µm.
65. The fiber according to one or more of items 62 to 64, wherein:
   (i) the fiber shell but not the fiber core is crosslinked or solidified; or
   (ii) the complete fiber is crosslinked or solidified, preferably comprising voids allowing the cells to grow and/or infiltrate into the voids.
66. The fiber according to one or more of items 62 to 65, wherein the fiber is a core-shell hollow fiber, preferably wherein the fiber shell is crosslinked or solidified not the fiber core, optionally the shell comprising a crosslinked polymer matrix.
67. The fiber according to item 66, having one or more of the following characteristics:
   (i) the core comprises the microcarrier and cells, preferably the microcarrier with the cells attached thereto;
   (ii) the microcarrier is provided at a concentration of least 0.001% (v/v) microcarrier related to total core volume, preferably at least 1% (v/v) or 10% (v/v) microcarrier related to total core volume;
   (iii) the fiber shell has a thickness selected from the range of 1 µm to 5,000 µm or 1 µm to 2,000 µm, preferably 5 µm to 1,000 µm, more preferably 10 µm to 300 µm or 20 µm to 100 µm; and/or
   (iv) the fiber core has a diameter selected from the range of 25 µm to 10,000 µm or 25 µm to 5,000 µm, preferably 100 µm to 1,000 µm, more preferably 200 µm to 700 µm or 200 µm to 400 µm;
68. The fiber according to item 66 or 67, wherein the core of the core-shell hollow fiber comprises voids, preferably having one or more of the following characteristics:
   (i) the voids are at least partially filled with a liquid, such as a cell culture medium;
   (ii) the voids provide sufficient space for the cells to proliferate, grow, and/or differentiate into the voids; and/or
   (iii) at least 30% (v/v) of the core volume are voids, preferably at least 40% (v/v) or at least 50% (v/v).
69. A cell-containing fiber, preferably being edible, produced by the method according to one or more of items 1 to 61, wherein the microcarrier and/or the crosslinked or solidified at least part of the fiber are dissolved or degraded.
70. The fiber according to item 69, wherein the cells of the fiber were cultured, preferably wherein the cells have produced an extracellular matrix, wherein preferably the extracellular matrix replaces the dissolved or degraded microcarrier and/or the crosslinked or solidified at least part of the fiber.
71. An edible, cell-containing fiber, preferably wherein the fiber is a core-shell hollow fiber, for use as artificial meat.
72. The fiber for use according to item 71, wherein the fiber is a fiber according to one or more of items 62 to 68 or 69 or 70, preferably produced by a method according to one or more of items 1 to 61.
73. The fiber for use according to item 71 or 72, wherein the artificial meat resembles meat selected from one or more of bovine meat, galline meat, chicken meat, bird meat, goat meat, deer meat, rabbit meat, kangaroo meat, porcine meat, fish meat and/or ovine meat.
74. The fiber for use according to one or more of items 71 to 73, wherein the fiber is collected or assembled for forming a fiber construct.
75. A spinning system for producing a cell-containing core-shell hollow fiber (4), wherein the fiber comprises a microcarrier and cells, preferably a microcarrier with cells interacting therewith, the system comprising:
   (i) a first container (1) for holding a microcarrier;
   (ii) a second container (2) for holding a composition comprising a compound capable of crosslinking or solidifying;
   (iii) a core-shell nozzle (3) for extruding the microcarrier through the core nozzle and extruding the composition through the shell nozzle; and
   (iv) a collector for collecting the cell-containing core-shell hollow fiber;
   wherein the first container (1) further is for holding the cells and/or the system comprises a further container for holding the cells.
76. The spinning system according to item 75, wherein the core-shell nozzle (3) has:
   - a core diameter selected from the range of 25 µm to 700 µm, preferably 100 µm to 500 µm; and/or
   - a shell diameter selected from the range of 100 µm to 5,000 µm, preferably 500 µm to 2,000 µm.
77. The spinning system according to item 75 or 76, wherein the system further comprises one or more of the following:
   - a further container for holding a non-solvent or crosslinking composition;
   - a source of irradiation, preferably irradiation with light, such as NIR, visible or UV light;
   - a temperature control unit;
   - a bioreactor for culturing the fiber; and/or
   - means for automation, such as a robotic arm or moving stage.
78. The spinning system according to one or more of items 75 to 77, wherein the collector for collecting the fiber is configured to perform a rotational, linear, angular, vibrational, and/or controlled or random movement, preferably a rotational movement, such as a rotating drum (10).
79. A method for producing artificial meat comprising:
   (I) producing one or more edible, cell-containing fiber(s), preferably wherein the fiber(s) are core-shell hollow fiber(s) and/or wherein the fiber(s) are fiber(s) according to one or more of items 62 to 68 or 69 or 70, preferably produced by a method according to one or more of items 1 to 61; and
   (II) collecting and/or assembling the one or more fiber(s) for forming a three-dimensional construct.
80. The method according to item 79, wherein collecting and/or assembling in step (II) further comprises stretching the one or more fiber(s), wherein preferably stretching comprises stretching the one or more fibers through rotational movement of a collector, such as a rotating drum.
81. The method according to item 79 or 80, wherein collecting and/or assembling in step (II) comprises assembling the one or more fiber(s) longitudinally predominantly in one direction, preferably assembling essentially parallel.
82. The method according to one or more of items 79 to 81, wherein step (I) comprises producing a plurality of edible, cell-containing fibers.
83. The method according to one or more of items 79 to 82, wherein the method further comprises step (III) culturing the cells of the three-dimensional construct.
84. The method according to item 83, wherein step (III) has one or more of the following characteristics:
   - culturing is performed for at least 24 h, preferably at least 48 h, more preferably at least 72 h;
   - culturing is performed at at least 25°C, preferably at least 30°C, more preferably at least 35°C, such as 36°C, 37°C, 38°C, 39°C, 40°C, or 41°C;
   - culturing is performed by providing a cell culture medium in which the three-dimensional construct is immersed or in contact with medium;
   - during culturing step (III) the cells proliferate, grow and/or differentiate, preferably wherein any voids of the one or more fiber(s) provide space for the cells to proliferate, grow, and/or differentiate;
   - during culturing step (III) the cells produce an extracellular matrix; and/or
   - during culturing step (III) the cells degrade the microcarrier and/or the crosslinked or solidified at least part of the fiber.
85. The method according to one or more of items 79 to 84, wherein the method has one or more of the following characteristics:
   (i) it comprises a step of dissolving or degrading the microcarrier, preferably after step (II), more preferably after or during a step of culturing the cells of the fiber(s);
   (ii) it comprises a step of dissolving or degrading the crosslinked or solidified at least part of the fiber(s), preferably after step (II), more preferably after or during a step of culturing the cells of the fiber(s);
   (iii) it comprises after step (II), preferably after or during a step of culturing the cells of the fiber(s), a step of dissolving or degrading the microcarrier and the crosslinked or solidified at least part of the fiber(s), such as a shell of core-shell hollow fiber(s).
   (iv) it comprises adding a degrading agent, preferably an enzymatic or chelating degrading agent for dissolving or degrading the microcarrier and/or the crosslinked or solidified at least part of the fiber(s); and/or
   (v) it comprises a step of culturing the cells of the fiber(s), wherein throughout culture the cells dissolve or degrade the microcarrier and/or the crosslinked or solidified at least part of the fiber(s).
86. Artificial meat comprising assembled and/or collected one or more edible, cell-containing fiber(s), preferably wherein the fiber(s) are core-shell hollow fiber(s) and/or wherein the fiber(s) are fiber(s) according to one or more of items 62 to 68 or 69 or 70, preferably produced by a method according to one or more of items 1 to 61.
87. The artificial meat according to item 86, wherein the artificial meat is produced by a method according to one or more of items 79 to 85.

Throughout the description, where methods, fibers, systems, compositions or uses are described as having, including, or comprising specific components or steps, it is contemplated that, additionally, there are methods, fibers, systems, compositions or uses of the present invention that consist essentially of, or consist of, the recited components or steps.

In the application, where an element or component is said to be included in and/or selected from a list of recited elements or components, it should be understood that the element or component can be any one of the recited elements or components, or the element or component can be selected from a group consisting of two or more of the recited elements or components.

Terms "a" and "an" and "the" and similar reference used in the context of describing the invention (especially in the context of the claims or items) are to be construed to cover both the singular and the plural, unless otherwise indicated herein or clearly contradicted by context.

The use of the term "include," "includes," "including," "have," "has," "having," "contain," "contains," or "containing," including grammatical equivalents thereof, should be understood generally as openended and non-limiting, for example, not excluding additional unrecited elements or steps, unless otherwise specifically stated or understood from the context.

Where the use of the term "about" or "approximately" is before a quantitative value, the present invention also includes the specific quantitative value itself, unless specifically stated otherwise. As used herein, the term "about" refers to a ±10% variation from the nominal value unless otherwise indicated or inferred.

Also as used herein, "and/or" refers to and encompasses any and all possible combinations of one or more of the associated listed items, as well as the lack of combinations when interpreted in the alternative ("or"). The use of the alternative (e.g., "or") should be understood to mean either one, both, or any combination thereof of the alternatives.

All citations are hereby incorporated by reference.

All individual embodiments and aspects as disclosed herein can be combined with each other within the framework and context of the present disclosure. It will be understood that the embodiments disclosed herein are only exemplary, and that any feature presented for a particular exemplary embodiment may be used with the present disclosure on its own or in combination with any feature presented for the same or another particular exemplary embodiment and/or in combination with any other feature not mentioned. It will further be understood that any feature presented for an example embodiment in a particular category may also be used in a corresponding manner in an example embodiment of any other category.

### EXAMPLES

It should be understood that the following examples are for illustrative purpose only and are not to be construed as limiting this invention in any manner. The following examples demonstrate the advantages of the method of the present invention, including successful formation of cell-containing fibers suitable for producing artificial meat. Specifically, the generated fibers allow for cells to be seeded with high accuracy and defined seeding density but at the same time provide enough space for the cells to mature into tissue. In addition, the fiber geometry achieves aligned tissue formation along the longitudinal fiber axis, mimicking native muscle strands. Furthermore, the cells within the fiber can be adequately supports by nutrients and oxygen, indicating excellent mass transport. By producing the fibers and collecting these using a rotating drum, followed by in vitro culture, the strands can recapitulate the highly organized aligned structure of muscle tissue, demonstrating the suitability for producing improved artificial meat.

### Materials and Methods

### Preparation of the composition comprising a compound capable of crosslinking or solidifying

In the Examples, alginate was used as compound for crosslinking or solidifying the fiber. Alginate (sodium alginate, Sigma-Aldrich) was dissolved in water with a concentration of 5 wt%.

### Preparation of the cells and microcarrier

As microcarriers ("MCs") the dextran based Cytodex1 (Cytodex^{®} 1 microcarrier beads, Sigma-Aldrich)) was used. According to the manufacturer information, Cytodex1 has positively charged N, N-diethylaminomethyl groups. The particle size distribution ranges from 140 to 200 µm. The MCs were swelled for 24 h in PBS, washed with Ca2+/Mg2+-free phosphate-buffered saline and autoclaved at 115 °C for 15 min. Before use, MCs were rinsed with DEMEM growth medium. The myoblast cell line C2C12 was used for providing the cells capable of interacting with the microcarriers. MCs (300 µl, 15 mg/ml) was given to a standard 24 culture well plate and inoculated with a cell density of 2 × 10⁵ cells (20 µL). After 20 min of incubation, DEMEM media was added and well plates were incubated for seven days at 37 °C and 5 % CO2 before spinning.

### Spinning process for core-shell hollow fibers

The fabrication of core-shell hollow fibers involved a meticulous wet spinning process utilizing specific materials and controlled parameters. **Fig. 1** shows a schematic representation of the spinning process and the formed core-shell hollow fiber. The wet fiber spinning process was executed using a customdesigned spinning setup (see **Fig. 2**), wherein the alginate solution was extruded through a core-shell nozzle. The nozzle had a core diameter of 300 µm and a shell diameter of 1 mm. The shell of the fibers was made of the alginate solution at a flow rate of 20 mL/hr, while the core was filled with a bore solution consisting of MCs suspended DMEM cell cultured medium at a flow rate of 10 mL/hr. As a proof-of-concept MCs used in this experiment were Cytodex1 (dextran-based MC).

The coagulation bath, comprising a CaCl2 solution, served as the medium for the solidification and stabilization of the hollow structure during wet fiber spinning. The entire process was conducted under controlled environmental conditions (Temp= 27C, RH=47%) to ensure reproducibility and reliability of the experimental outcome. After the fiber spinning in a sterile condition, hollow fiber composites were cultured at 37 °C and 5% CO2.

### Analysis of the fibers

Using microscopy techniques, the fibers were analyzed to determine their diameter, morphology, and the structure of encapsulated cells. Brightfield microscopy was employed to measure the fiber diameter and observe the overall morphology, including features such as formation of hollow fibers and encapsulation of MCs within the fiber's core. This technique provided detailed images of the fibers under bright illumination, where MC were dyed for better visualization. Fluorescence microscopy was utilized for visualizing fluorescently labeled cells encapsulated within the fibers. This method enabled the observation of cell morphology, distribution, and interaction with the fiber and the MC. By staining the cells with fluorescent dyes that bind to specific cellular components, such as the cell membrane (cytoskeleton) or nucleus, we obtained detailed insights into the structure of the encapsulated cells such as cell distribution on MCs and elongation in the direction of fiber's long axis. Image analysis software such as imaged was used to process the microscopy images.

### Example 1: Cell-containing, core-shell hollow fibers are suitable for producing artificial meat

Example 1 demonstrates that the method according to the present disclosure allows for generating superior cell-containing fibers suitable for producing artificial meat with organized structure. Specifically, in Example 1 core-shell hollow fibers were produced as disclosed above. Specifically, an alginate solution (5 wt%) was used as shell material and C2C12 cells precultured on Cytodex1 dextran microcarriers were dispersed in DMEM cell culture medium were used as core. After extrusion through the core-shell nozzle, the cell-containing fiber was at least partially crosslinked (i.e. the fiber shell) and collected using a rotating drum. The produced cell-containing fiber was then cultured for 10 days, followed by microscopic analysis. The results can be seen in **Figs. 3** and **4****-8.**

**Fig. 3** shows a produced core-shell hollow fiber comprising MCs at day 0 right after spinning (left side) and after 10 days in culture (right side). The core of the fiber contains the microcarriers and cells interacting therewith, whereas shell comprises the crosslinked alginate. It can also be seen that after culture, the cells grow on top and in the space between the MCs. They also fill up larger empty spaces within the core and form linear muscle tissue parallel to the long axis of the core-shell hollow fiber.

Furthermore, post fabrication, the collected hollow fibers underwent a thorough analysis to understand the distribution of MCs within the core. The concentration of MCs in the bore solution played a critical role in determining the packing density within the core. Higher concentrations resulted in a more densely packed distribution of nanoparticles, showcasing the tunability of the fabrication process. Importantly, without necessitating intricate on-demand on-cell systems or other degradation mechanisms proposed in prior studies, our approach allows ample space for cell growth, migration, and spreading in a 3D environment. Surprisingly, we have observed that cells can grow further in the empty space of hollow fibers where there is no MC present. This enables populating the whole empty space of the core area with cells (**Fig. 3**). Another intriguing result that we observed was the ability of the cells growing parallel to the long axis direction of the fibers. This can further be seen in **Figs. 4-****8,** showing exemplary fluorescence microscopy images of the core-shell hollow fiber after culturing C2C12 cells attached to MCs within the fibers. The images originate from different magnifications and parts of one core shell hollow fiber. As visible in the microscopic images, the cells populate the empty space between the MCs and form filamentous elongated structures, indicating that the cells produce an own aligned ECM. This clearly indicates formation of muscle fibers, that are suitable to be used for producing artificial meat.

### Example 2: Prophetic exemplification of a cell-containing fiber with complete crosslinking

In Example 2, the cell-containing fiber is fully crosslinked or solidified compared to the core-shell hollow fiber as disclosed in Example 1. In Example 2, a hollow fiber structure is formed by phase separation.

Here alginate polymer solution is prepared in water (as solvent) and it is extruded in a non-solvent such as ethanol to induce phase separation. The addition of the non-solvent induces a phase separation, causing the polymer to precipitate out of the solution. This results in the formation of a polymer-rich outer layer and a non-solvent-rich inner core, creating the hollow structure of the fiber. The rapid precipitation near the fiber surface forms a skin layer, acting as a barrier to the non-solvent. Post-treatment steps like cross-linking may be applied to enhance stability and mechanical strength of hollow fibers.

### Example 3: Prophetic exemplification of using cell containing fiber to produce artificial meat

In Example 3, the cell-containing fiber of Example 1 or 2 is used in order to form a macroscopic structure for producing artificial meat.

Cells are isolated from a small tissue sample or biopsy from animal and cultured in a bioreactor to increase their numbers on MCs. The bioreactor provides an optimal environment with nutrients and growth factors for cell proliferation and attachment to the MCs. Once the desired cell density is achieved, the cells are spun into cell containing fibers according to one of the previous examples.

The cell-laden fibers are then collected on a rotating drum system, where the rotation of the drum allows for controlled alignment and stretching of the fibers. This step not only aids in scalability as large quantities of fibers can be collected efficiently, but also helps mimic the natural alignment found in muscle tissue. Next, the collected cell-laden fibers are transferred to a perfusion bioreactor for further cultivation. Here, the fibers are immersed in a nutrient-rich growth medium that flows through them, providing a continuous supply of nutrients and oxygen to the cells. The space between MCs enables cells growth and expansion within the fibers. Over 1-2 weeks, the cells in the fibers proliferate and mature and/or differentiate into muscle-like cells. The fibers act as scaffolds, guiding the cells to grow in parallel along the length of the fibers. This process results in the formation of aligned muscle fibers, closely mimicking the texture and structure of natural meat. As the cells continue to grow and mature producing their own ECM, MCs and fiber shell degrades using an enzymatic or chelating method. This results in artificial meat tissue development which possess natural texture and flavor characteristic.

### Conclusions

The Examples demonstrate that the methods, materials, and devices according to the present invention allow for producing cell-containing fibers that can be used for artificial meat. It is shown that by using the microcarriers with the cells capable of interacting with the microcarriers, in particular by attaching thereto, the cells can be delivered and thus seeded in a controlled manner within the fiber. Moreover, by providing a cell-containing fiber containing voids, the cells have sufficient space to proliferate, and form aligned muscle tissue. Hence, the method enables tissue maturation indicating that adequate nutrition and sufficient oxygen supply to the cells within the fiber is possible. By designing the materials of the shell and microcarriers to degrade overtime, full muscle tissue strands can be generated essentially containing no additional materials than cells and produced ECM. Moreover, by using the herein disclosed rotating drum, the cell-containing fibers can be collected in an aligned manner allowing for assembly of macroscopic 3D structures up to large scale, closely resembling the organized macroscopic structure of native muscle.

## Claims

1. A method for producing a cell-containing, preferably edible, fiber comprising:
(a) providing
- a microcarrier,
- cells capable of interacting with the microcarrier, and
- a composition comprising a compound capable of crosslinking or solidifying;
(b) spinning the microcarrier, the cells and the composition to generate a fiber; and
(c) crosslinking or solidifying at least a part of the fiber.

2. The method according to claim 1, wherein step (b) comprises spinning the microcarrier and the cells, preferably the microcarrier with the cells attached thereto, and the composition to form a core-shell hollow fiber, preferably by using a core-shell nozzle, wherein
- the microcarrier and the cells are extruded through the core nozzle, and
- the composition is extruded through the shell nozzle.

3. The method according to claim 2, wherein step (c) comprises crosslinking or solidifying the fiber shell and preferably not the fiber core, preferably the core-shell hollow fiber having following characteristics:
(i) the fiber shell has a thickness selected from the range of 1 µm to 5,000 µm or 1 µm to 2,000 µm, preferably 5 µm to 1,000 µm, more preferably 10 µm to 300 µm or 20 µm to 100 µm; and/or
(ii) the fiber core has a diameter selected from the range of 25 µm to 10,000 µm or 25 µm to 5,000 µm, preferably 100 µm to 1,000 µm, more preferably 200 µm to 700 µm or 200 µm to 400 µm.

4. The method according to claim 2 or 3, wherein the core of the core-shell hollow fiber comprises voids, preferably wherein the voids are at least partially or essentially fully filled with a liquid wherein the voids provide sufficient space for the cells to proliferate, grow, and/or differentiate into the voids, optionally wherein
(i) at least 1% (v/v) of the core volume are voids, preferably at least 1% (v/v), at least 2% (v/v), at least 5% (v/v), at least 10% (v/v), at least 15% (v/v), at least 20% (v/v), at least 25% (v/v), or at least 30% (v/v) of the core volume are voids, more preferably at least 40% (v/v) or at least 50% (v/v) of the core volume are voids; and/or
(ii) the microcarrier is provided at a concentration of least 0.001% (v/v) microcarrier related to total core volume, preferably at least 1% (v/v) or 10% (v/v) microcarrier related to total core volume.

5. The method according to one or more of claims 1 to 4, wherein the cells have one or more of the following characteristics:
(i) they are eukaryotic cells, preferably, animal cells,
(ii) they are one or more cell types found in meat, preferably one or more cell types found in bovine meat, galline meat, chicken meat, bird meat, goat meat, deer meat, rabbit meat, kangaroo meat, porcine meat, fish meat and/or ovine meat;
(iii) the cells are one or more cell types found in an animal, preferably one or more cell types isolated or derived from bovine cells, galline cells, chicken cells, bird cells, goat cells, deer cells, rabbit cells, kangaroo cells, porcine cells, fish cells and/or ovine cells;
(iv) they are selected from one or more of the group of myocytes, adipocytes, fibroblasts, chondrocytes, hematopoietic cells, muscle satellite cells, mesenchymal stem/stromal cells, fibro/adipogenic progenitor cells, endothelial cells, and pluripotent stem cells, such as induced pluripotent stem cells or embryonic stem cells;
(v) the cells comprise at least a myocyte and/or a muscle satellite cell, optionally further comprising adipocytes, mesenchymal stem/stromal cells, fibro/adipogenic progenitor cells, endothelial cells, and/or pluripotent stem cells;
(vi) they are alive, apoptotic and/or dead;
(vii) they are differentiated cells, non-differentiated cells, pluripotent cells, multipotent cells, reprogrammed cells, or a combination thereof; and/or
(viii) they are capable of interacting with the microcarrier by being capable of (i) attaching to the microcarrier and/or (ii) sensing chemical or biochemical cues originating from the microcarrier.

6. The method according to one or more of claims 1 to 5, wherein step (a) further comprises providing a microcarrier without cells attached thereto, preferably wherein the volume or mass ratio of provided microcarrier with cells attached thereto to provided microcarrier without cells attached thereto is between 10:1 to 1:1000, preferably 2:1 to 1:100, more preferably 1:1 to 1:100 or 1:1 to 1:50.

7. The method according to one or more of claims 1 to 6, wherein the microcarrier
- comprises a surface that is suitable for the cells to attach thereto, preferably suitable for anchorage-dependent cells, and/or wherein the microcarrier comprises a cell-adhesive surface;
- is degradable or dissolvable, such as enzymatically degradable and/or degradable or dissolvable by a chelating agent;
- has a size selected from 10 µm to 1,000 µm, preferably 50 µm to 500 µm, more preferably 100 µm to 300 µm; and/or
- is dispersed in a liquid, preferably the liquid being non-crosslinked, partially crosslinked or crosslinked, more preferably the liquid being non-crosslinked or partially crosslinked, most preferably the liquid comprises is suitable for storing and/or culturing the cells at least for a short time, such as at least for 15 min, 30 min, 45 min or 60 min, preferably more than 60 min, such as a cell culture medium.

8. The method according to one or more of claims 1 to 7, wherein spinning step (b) is performed by a process selected from
(i) melt spinning, extrusion spinning or direct spinning; or
(ii) solution spinning, such as dry spinning, wet spinning, gel spinning or electrospinning; or
(iii) a combination of any process of (i) and/or (ii); or
(iv) preferably solution spinning, more preferably wet spinning.

9. The method according to one or more of claims 1 to 8, wherein the method further comprises a step of culturing the cells of the fiber, preferably after step (c), optionally wherein the method has one or more of the following characteristics:
(i) it comprises a step of dissolving or degrading the microcarrier, preferably after step (c), more preferably after or during a step of culturing the cells of the fiber;
(ii) it comprises a step of dissolving or degrading the crosslinked or solidified at least part of the fiber, preferably after step (c), more preferably after or during a step of culturing the cells of the fiber;
(iii) it comprises after step (c), preferably after or during a step of culturing the cells of the fiber, a step of dissolving or degrading the microcarrier and the crosslinked or solidified at least part of the fiber, such as a shell of a core-shell hollow fiber;
(iv) it comprises adding a degrading agent, preferably an enzymatic or chelating degrading agent for dissolving or degrading the microcarrier and/or the crosslinked or solidified at least part of the fiber; and/or
(v) it comprises a step of culturing the cells of the fiber, wherein throughout culture the cells dissolve or degrade the microcarrier and/or the crosslinked or solidified at least part of the fiber.

10. A cell-containing fiber, preferably being edible, comprising a microcarrier with cells interacting therewith, wherein at least a part of the fiber is crosslinked or solidified.

11. The fiber according to claim 10, wherein the fiber is a core-shell hollow fiber, preferably wherein the fiber shell is crosslinked or solidified not the fiber core and the core of the core-shell hollow fiber comprises voids, preferably having one or more of the following characteristics:
(i) the voids are at least partially filled with a liquid, such as a cell culture medium;
(ii) the voids provide sufficient space for the cells to proliferate, grow, and/or differentiate into the voids; and/or
(iii) at least 30% (v/v) of the core volume are voids, preferably at least 40% (v/v) or at least 50% (v/v).

12. A cell-containing fiber, preferably being edible, produced by the method according to one or more of claims 1 to 9, preferably wherein at least part the microcarrier and/or the crosslinked or solidified fiber are dissolved or degraded, optionally wherein the cells have produced an extracellular matrix.

13. An edible, cell-containing fiber, preferably wherein the fiber is a core-shell hollow fiber and/or a fiber according to one or more of claims 10 to 12, for use as artificial meat.

14. A method for producing artificial meat comprising:
(I) producing one or more edible, cell-containing fiber(s), preferably wherein the fiber(s) are core-shell hollow fiber(s) and/or wherein the fiber(s) are fiber(s) according to one or more of claims 10 to 12, preferably produced by a method according to one or more of claims 1 to 9; and
(II) collecting and/or assembling the one or more fiber(s) for forming a three-dimensional construct.

15. Artificial meat comprising assembled and/or collected one or more edible, cell-containing fiber(s), preferably wherein the fiber(s) are core-shell hollow fiber(s) and/or wherein the fiber(s) are fiber(s) according to one or more of claims 10 to 12, preferably produced by a method according to one or more of claims 1 to 9.
